# EUROPEAN PATENT APPLICATION

(11) **EP 3 971 163 A1**
(43) Date of publication of application: **23.03.2022**
(21) Application number: 20804838.9
(22) Date of filing: 11.05.2020
(51) Int. Cl.: C07C 63/49, C07C 63/66, C07C 63/72, C07C 63/74, C07F 1/08, C07F 3/02, C07F 3/06, C07F 5/06, C07F 11/00, C07F 7/00, B01D 53/02, B01J 20/22, B01J 20/26

(54) **METAL-ORGANIC STRUCTURAL BODY**

(30) Priority: 13.05.2019 JP 2019090662; 24.09.2019 JP 2019172510
(71) Applicant: Rikkyo Educational Corporation, Tokyo 171-8501 (JP); Nippon Soda Co., Ltd., Tokyo 100-8165 (JP)
(72) Inventor: MINOURA, Mao, Tokyo 171-8501 (JP); SUGAMATA, Koh, Tokyo 171-8501 (JP); YANAGISAWA, Daichi, Tokyo 171-8501 (JP); IIHAMA, Teruyuki, Tokyo 100-8165 (JP)
(74) Representative: Wibbelmann, Jobst
(86) International application number: PCT/JP2020/018825
(87) International publication number: WO 2020/230756

(57) **Abstract**

An object of the present invention is to provide a metal-organic framework capable of adsorbing a gas such as a hydrogen molecule or carbon dioxide at a practical level. The metal-organic framework is used for adsorbing a gas such as hydrogen or carbon dioxide and comprises a multivalent metal ion and a carboxylate ion of formula [I] [wherein in formula [I], X¹ to X³ each independently represent a functional group of formula [II] (wherein in formula [II], Z is a single bond or a multivalent linking group, k is an integer of 1 to 4, and * is the position at which a bond is formed with a benzene ring) ; and Y¹ and Y² each independently represent a hydrogen atom, a halogeno group, a C1-6 alkyl group or the like, provided that when the multivalent metal ion is a trivalent metal ion, Y¹ and Y² each independently represent a halogeno group, a C1-6 alkyl group or the like], wherein the carboxylate ion and the multivalent metal ion are bound to each other.

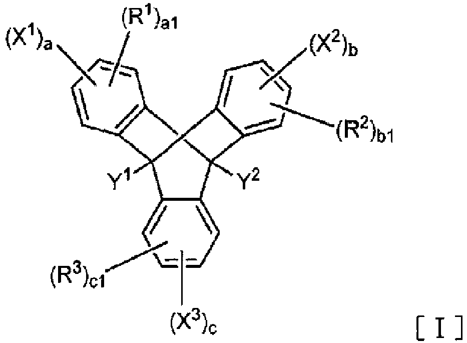

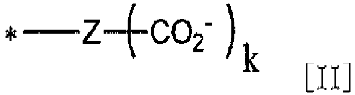

## Description

### Technical Field

The present invention relates to a novel metal-organic framework and a gas adsorption method using the metal-organic framework.

The present application claims priority of Japanese Patent Application No. 2019-90662 filed on May 13, 2019 and Japanese Patent Application No. 2019-172510 filed on September 24, 2019, the contents of which are hereby incorporated by reference.

### Background Art

A metal-organic framework (hereinafter sometimes referred to as "MOF") is a substance in a solid state that has a macromolecular structure with a space inside (that is, pores) by combining metal ions and a crosslinkable organic ligand that connects them. The metal-organic framework has been of great interest for more than a decade as a porous material with such a function as gas storage or separation.

As the crosslinkable organic ligand, an oxygen donor ligand and a nitrogen donor ligand have been often used.

The MOF obtained from triptycene-9,10-dicarboxylic acid having a triptycene skeleton as a crosslinkable organic ligand, zinc nitrate and dipyridyl has been known to adsorb a low-molecular compound such as diacetylene or amyl acetate (see Patent Document 1).

It has been also known that hexakis(4-carboxyphenyl)triptycene in which two 4-carboxyphenyl groups are symmetrically arranged on each benzene ring of triptycene forms, via a hydrogen bond between the carboxylate groups, a framework having a macromolecular structure having pores in the molecule and the framework adsorbs a nitrogen molecule and a carbon dioxide molecule (see Non-patent Document 1).

It has further been known that the MOF comprising hexakis(4-carboxyphenyl)triptycene in which two 4-carboxyphenyl groups are symmetrically arranged on each benzene ring of triptycene as an organic ligand and trivalent metal ions, Fe³⁺, Cr³⁺ and Sc³⁺ has a BET specific surface area of 3580 m²/g to 4280 m²/g and adsorbs a water molecule (see Non-patent Document 2).

Meanwhile, with the advent of a hydrogen energy-based society, researches on adsorption of hydrogen with MOF also have been actively carried out. For example, it has been known that a MOF consisting of a tricarboxylic acid ion and a zinc cluster ion having the following structure can adsorb 6% by mass of hydrogen based on the total amount of MOF at room temperature and 90 atm (see Patent Document 2).

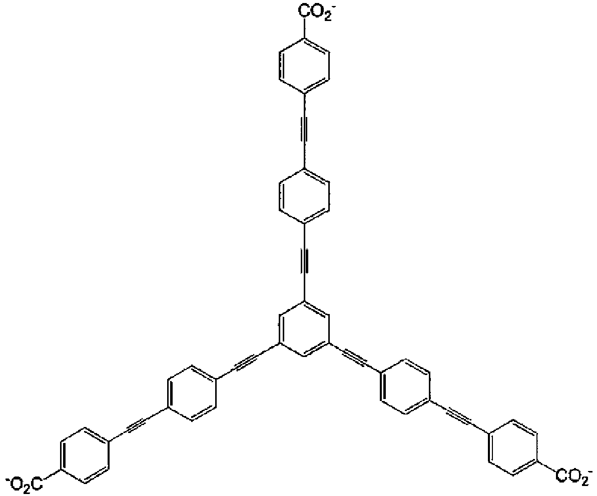

### Prior Art Documents

### Patent Documents

Patent Document 1: International Publication WO 2009/098001
Patent Document 2: Japanese unexamined Patent Application Publication No. 2018-058814

### Non-patent Documents

Non-patent Document 1: J. F. Stoddart, et al, Angew. Chem. Int. Ed., 2019, 58, 1664-1669
Non-patent Document 2: J. F. Stoddart, et al, J. Am. Chem. Soc., 2019, 141, 2900-2905

### Summary of the Invention

### Objects to be Solved by the Invention

Any MOF capable of adsorbing a gas such as a hydrogen molecule, a nitrogen molecule or carbon dioxide at a practical level has not yet been known.

An object of the present invention is to provide a novel MOF capable of adsorbing a gas such as a hydrogen molecule, a nitrogen molecule or carbon dioxide.

### Means to Solve the Object

As a result of diligent studies to solve the above problems, the present inventors have found that an MOF obtained by using an organic ligand having a three-dimensional space with triptycene as a mother nucleus and a multivalent metal ion has a high capacity to adsorb a gas such as a hydrogen molecule, a nitrogen molecule or carbon dioxide, and have completed the present invention.

That is, the present invention is specified by the following items that follows:
[1] A metal-organic framework comprising a multivalent metal ion and a carboxylate ion of formula [I]: 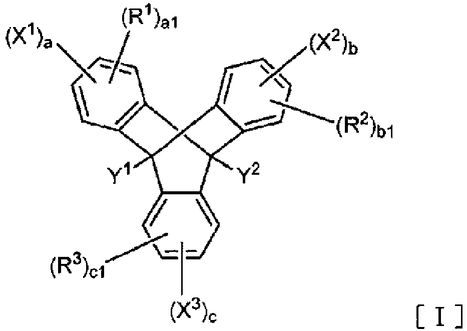 [wherein in formula [I],
   X¹ to X³ each independently is a functional group of formula [II]: 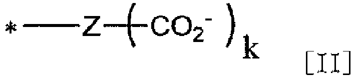 (wherein in formula [II], Z is a single bond or a multivalent linking group, k is an integer of 1 to 4, and * is the position at which a bond is formed with a benzene ring and has also the same meaning in the following);
   R¹ to R³ each independently is a hydrogen atom, a C1-6 alkyl group, a C3-8 cycloalkyl group, a C6-10 aryl group, a 3 to 6-membered heterocyclyl group, a C1-6 alkoxy group, a C6-10 aryloxy group, a heteroaryloxy group, a halogeno group, C1-6 haloalkyl group, a C6-10 haloaryl group, a C1-6 haloalkoxy group, C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [III]: 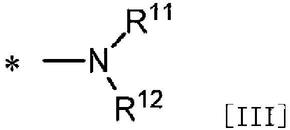 (wherein in formula [III], R¹¹ and R¹² each independently is a hydrogen atom, a C1-6 alkyl group, a C6-10 aryl group, a C1-6 alkylcarbonyl group or a C6-10 arylcarbonyl group);
   a is the number of X¹ and is 0, 1, 2, 3 or 4, and when a is 2 or more, a plurality of X¹s is the same as or different from each other;
   a1 is the number of R¹ and is 0, 1, 2, 3 or 4, and when a1 is 2 or more, a plurality of R¹s is the same as or different from each other;
   b is the number of X² and is 0, 1, 2, 3 or 4, and when b is 2 or more, a plurality of X²s is the same as or different from each other;
   b1 is the number of R² and is 0, 1, 2, 3 or 4, and when b1 is 2 or more, a plurality of R²s is the same as or different from each other;
   c is the number of X³ and is 0, 1, 2, 3 or 4, and when c is 2 or more, a plurality of X³s is the same as or different from each other; and
   c1 is the number of R³ and is 0, 1, 2, 3 or 4, and when c1 is 2 or more, a plurality of R³s is the same as or different from each other;
   provided that a + b + c is 2 or more; and
   Y¹ and Y² each independently is a hydrogen atom, a halogeno group, a C1-6 alkyl group or a C1-6 alkoxy group, provided that when the multivalent metal ion is a trivalent metal ion, Y¹ and Y² each independently is a halogeno group, a C1-6 alkyl group or a C1-6 alkoxy group];
   wherein the carboxylate ion and the multivalent metal ion are bound to each other.
[2] The metal-organic framework according to [1], wherein the multivalent metal ion is an ion of at least one metal selected from the group consisting of Groups 2 to 13 metals in the periodic table of elements.
[3] The metal-organic framework according to [1] or [2], wherein the multivalent metal ion is an ion of at least one metal selected from Zn, Fe, Co, Ni, Cu, Zr and Mg.
[4] The metal-organic framework according to any one of [1] to [3], wherein the multivalent linking group shown by Z in formula [I] is a divalent or trivalent linking group.
[5] The metal-organic framework according to any one of [1] to [4], wherein the multivalent linking group shown by Z in formula [I] is: a linking group selected from a linking group A group consisting of divalent to tetravalent linking groups derived from an unsubstituted alkane or an alkane having a substituent (except for a carboxy group); divalent to tetravalent linking groups derived from unsubstituted ethene or ethene having a substituent (except for a carboxy group); an ethynylene group; divalent to tetravalent linking groups derived from an unsubstituted aryl or an aryl having a substituent (except for a carboxy group); divalent to tetravalent linking groups derived from an unsubstituted heteroaryl or a heteroaryl having a substituent (except for a carboxy group); and a combination thereof; a linking group selected from a linking group B group consisting of -O-, -S-, -S(O)-, -SO₂-, -C(=O)-, linking groups of following formulae [IV-1] to [IV-3]: 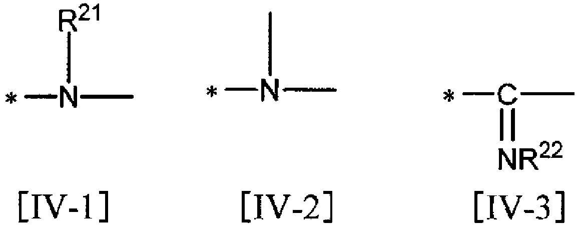 (wherein R²¹ and R²² each independently represent a hydrogen atom, a C1-6 alkyl group, a C6-10 aryl group, a C1-6 alkylcarbonyl group or a C6-10 arylcarbonyl group)
   and a combination thereof; or a combination of a linking group selected from the linking group A group and a linking group selected from the linking group B group, provided that the carboxylate ionic group (CO₂⁻) in formula [II] is bonded to the carbon atom in Z in formula [II].
[6] The metal-organic framework according to any one of [1] to [5], wherein in formula [I], formula [II] is formula [V-1] or formula [V-2]: 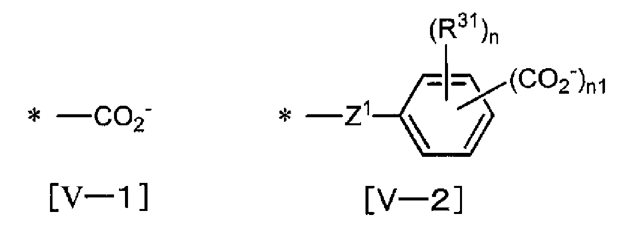 (wherein in formula [V-2],
   Z¹ is a single bond, -C=C-, -O-, -S-, -S(O)-, -SO₂- or -C(=O)-;
   R³¹ is a C1-6 alkyl group, a C3-8 cycloalkyl group, a C6-10 aryl group, a 3 to 6-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [VI]: 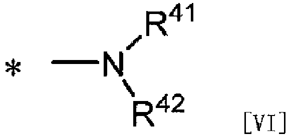 (wherein in formula [VI], R⁴¹ and R⁴² each independently represent a hydrogen atom, a C1-6 alkyl group, a C6-10 aryl group, a C1-6 alkylcarbonyl group or a C6-10 arylcarbonyl group);
   n is the number of R³¹ and is 0, 1, 2, 3 or 4, and when n is 2 or more, a plurality of R³¹s is the same as or different from each other; and
   n1 is the number of CO₂⁻ and is 1 or 2) .
[7] The metal-organic framework according to any one of [1] to [6], further comprising, as a constituent, an organic ligand other than the carboxylate ion of formula [I].
[8] A method for storing a gas, comprising a step of contacting a gas with the metal-organic framework according to any one of [1] to [7] to cause the gas to be adsorbed inside the metal-organic framework.

### Effect of the Invention

The use of the MOF of the present invention enables a hydrogen molecule and a carbon dioxide molecule to be adsorbed in a larger amount than a conventional one.

### Brief Description of Drawings

[Figure 1] Figure 1 shows a nitrogen adsorption isotherm at 77K of Metal-organic framework 1 obtained in Example 1.
[Figure 2] Figure 2 shows a hydrogen adsorption isotherm at 77K of Metal-organic framework 1 obtained in Example 1.

### Mode of Carrying Out the Invention

The metal-organic framework of the present invention is a metal-organic framework in which a multivalent metal ion and a carboxylate ionic group in an organic ligand of formula [I] are bound to each other.

The multivalent metal ion used in the present invention is preferably an ion of at least one metal selected from the group consisting of Groups 2 to 13 metals in the periodic table of elements, and preferably an ion of at least one metal selected from Zn, Fe, Co, Ni, Cu, Zr, Sc, Cr, Al and Mg. The multivalent metal ion is preferably a divalent or tetravalent metal ion, preferably an ion of at least one metal selected from Zn, Fe, Co, Ni, Cu, Zr and Mg, and more preferably an ion of at least one metal selected from Co, Ni, Cu, Zr and Zn.

These multivalent metal ions are provided in the form of various salts. Specific examples of the salts can include Zn(NO₃)₂•6H₂O, Zn(NO₃)₂•4H₂O, ZrCl₄, ZrOCl₂, Ni(NO₃)₂•6H₂O, Mg(NO₃)₂•6H₂O, Cu(NO₃)₂•xH₂O, Cu(NO₃)_{2•}2.5H₂O, Co(NO₃)₂•6H₂O, Cr(NO₃)₃•9H₂O, CrCl₃•6H₂O and Al(N0₃)ₑ•9HₑO.

The organic ligand used in the present invention comprises a carboxylate ion of formula [I].

In formula [I], X¹ to X³ each independently represent a functional group of formula [II]. In formula [II], Z is a single bond or a multivalent linking group.

The single bond means that the carboxylate ionic group (CO₂⁻) is directly bound to a benzene ring.

The multivalent linking group is a divalent or higher linking group and is not particularly limited as long as it can link a benzene ring and a carboxylate ionic group with each other. Among them, the multivalent linking group is preferably a divalent or trivalent linking group.

The structure of the multivalent linking group also not particularly limited as long as it can link a benzene ring and a carboxylate ionic group with each other. Particularly preferred examples of the multivalent linking group can include a linking group selected from a linking group A group consisting of divalent to tetravalent linking groups derived from an unsubstituted alkane or an alkane having a substituent (except for a carboxy group); divalent to tetravalent linking groups derived from unsubstituted ethene or ethene having a substituent (except for a carboxy group) ; an ethynylene group; divalent to tetravalent linking groups derived from an unsubstituted aryl or an aryl having a substituent (except for a carboxy group); divalent to tetravalent linking groups derived from an unsubstituted heteroaryl or a heteroaryl having a substituent (except for a carboxy group); and a combination thereof; a linking group selected from a linking group B group consisting of -O-, -S-, -S(O)-, -SO₂⁻, -C(=O)-, linking groups of following formulae [IV-1] to [IV-3]:

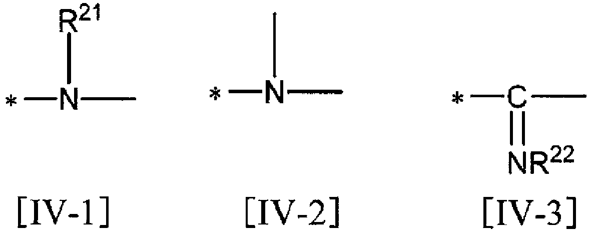

(wherein R²¹ and R²² each independently represent a hydrogen atom, a C1-6 alkyl group, a C6-10 aryl group, a C1-6 alkylcarbonyl group or a C6-10 arylcarbonyl group) and a combination thereof; or a combination of a linking group selected from the linking group A group and a linking group selected from the linking group B group, provided that the carboxylate ionic group (CO₂⁻) in formula [II] is bonded to the carbon atom in Z in formula [II].

As used herein, the term "unsubstituted" means that only the group of a mother nucleus is present. When only the name of the group of a mother nucleus is described without the term "substituted", it means an "unsubstituted" group unless otherwise specified.

The expression "having a substituent" is used synonymously with the term "substituted", and means that any hydrogen atom of the group of a mother nucleus is replaced with a functional group (substituent) having the same structure as or different structure from the structure of the mother nucleus. Therefore, the "substituent" is another functional group attached to the functional group of a mother nucleus. The substituent may be one or more. The two or more substituents are the same as or different from each other.

Such terms as "C1-6" indicate that the number of carbon atoms of the group of a mother nucleus is 1 to 6, or the like. This number of carbon atoms does not include the number of carbon atoms present in the substituent. For example, a butyl group having an ethoxy group as a substituent is classified as a C2 alkoxy-C4 alkyl group.

The "substituent" is not particularly limited as long as it is chemically acceptable and has the effect of the present invention. Examples of the group that can be a "substituent" include:
a C1-6 alkyl group such as a methyl group, an ethyl group, an n-propyl group, an i-propyl group, an n-butyl group, an s-butyl group, an i-butyl group, a t-butyl group, an n-pentyl group or an n-hexyl group;
a C2-6 alkenyl group such as a vinyl group, a 1-propenyl group, a 2-propenyl group (allyl group), a 1-butenyl group, a 2-butenyl group, a 3-butenyl group, a 1-methyl-2-propenyl group or a 2-methyl-2-propenyl group;
a C2-6 alkynyl group such as an ethynyl group, a 1-propynyl group, a 2-propynyl group (propargyl group), a 1-butynyl group, a 2-butynyl group, a 3-butynyl group or a 1-methyl-2-propynyl group;
a C3-8 cycloalkyl group such as cyclopropyl group, a cyclobutyl group, a cyclopentyl group, a cyclohexyl group or cubanyl group;
a C6-10 aryl group such as a phenyl group or a naphthyl group;
a C6 to 10 aryl-Cl to 6 alkyl group such as a benzyl group or a phenethyl group;
a 3 to 6-membered heterocyclyl group;
a 3 to 6-membered heterocyclyl-Cl to 6 alkyl group;
a hydroxyl group;
a C1-6 alkoxy group such as a methoxy group, an ethoxy group, an n-propoxy group, an i-propoxy group, an n-butoxy group, an s-butoxy group, an i-butoxy group or a t-butoxy group;
a C2-6 alkenyloxy group such as a vinyloxy group, an allyloxy group, a propenyloxy group or a butenyloxy group;
a C2-6 alkynyloxy group such as an ethynyloxy group or a propargyloxy group;
a C6-10 aryloxy group such as a phenoxy group or a naphthoxy group;
a C6-10 aryl-C1-6 alkoxy group such as a benzyloxy group or a phenethyloxy group;
a 5 to 6-membered heteroaryloxy group such as a thiazolyloxy group or a pyridyloxy group;
a 5 to 6-membered heteroaryl-C1-6 alkyloxy group such as a thiazolylmethyloxy group or a pyridylmethyloxy group;
a formyl group;
a C1-6 alkylcarbonyl group such as an acetyl group or a propionyl group;
a formyloxy group;
a C1-6 alkylcarbonyloxy group such as an acetyloxy group and a propionyloxy group;
a C6-10 arylcarbonyl group such as a benzoyl group;
a C1-6 alkoxycarbonyl group such as a methoxycarbonyl group, an ethoxycarbonyl group, an n-propoxycarbonyl group, an i-propoxycarbonyl group, an n-butoxycarbonyl group or a t-butoxycarbonyl group;
a C1-6 alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group or a t-butoxycarbonyloxy group;
a carboxy group;
a halogeno group such as a fluoro group, a chloro group, a bromo group or iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group or a 2,4,6-trichlorohexyl group;
a C2-6 haloalkenyl group such as a 2-chloro-1-propenyl group or a 2-fluoro-1-butenyl group;
a C2-6 haloalkynyl group such as a 4,4-dichloro-1-butynyl group, a 4-fluoro-1-pentynyl group or a 5-bromo-2-pentynyl group;
a C1-6 haloalkoxy group such as a trifluoromethoxy group, a 2-chloro-n-propoxy group or a 2,3-dichlorobutoxy group;
a C2-6 haloalkenyloxy group such as a 2-chloropropenyloxy group or a 3-bromobutenyloxy group;
a C1-6 haloalkylcarbonyl group such as a chloroacetyl group, a trifluoroacetyl group or a trichloroacetyl group;
an amino group;
a C1-6 alkyl-substituted amino group such as a methylamino group, dimethylamino group or a diethylamino group;
a C6-10 arylamino group such as an anilino group or a naphthylamino group;
a C6-10 aryl-C1-6 alkylamino group such as a benzylamino group or a phenethylamino group;
a formylamino group;
a C1-6 alkylcarbonylamino group such as an acetylamino group, a propanoylamino group, a butyrylamino group or an i-propylcarbonylamino group;
a C1-6 alkoxycarbonylamino group such as a methoxycarbonylamino group, an ethoxycarbonylamino group, an n-propoxycarbonylamino group or an i-propoxycarbonylamino group;
an unsubstituted aminocarbonyl group or an aminocarbonyl group having a substituent, such as an aminocarbonyl group, a dimethylaminocarbonyl group, a phenylaminocarbonyl group or an N-phenyl-N-methylaminocarbonyl group;
an imino-C1-6 alkyl group such as an iminomethyl group, a (1-imino)ethyl group or a (1-imino)-n-propyl group;
a substituted or unsubstituted N-hydroxyimino-C1-6 alkyl group such as an N-hydroxy-iminomethyl group, a (1-(N-hydroxy)-imino)ethyl group, a (1-(N-hydroxy)-imino)propyl group, an N-methoxy-iminomethyl group or a (1-(N-methoxy)-imino)ethyl group;
a C1-6 alkoxyimino group such as a methoxyimino group, an ethoxyimino group, an n-propoxyimino group, an i-propoxyimino group or an n-butoxyimino group;
an aminocarbonyloxy group;
a C1-6 alkyl-substituted aminocarbonyloxy group such as an ethylaminocarbonyloxy group or a dimethylaminocarbonyloxy group;
a mercapto group;
a C1-6 alkylthio group such as a methylthio group, an ethylthio group, an n-propylthio group, an i-propylthio group, an n-butylthio group, an i-butylthio group, an s-butylthio group or a t-butylthio group;
a C1-6 haloalkylthio group such as a trifluoromethylthio group or a 2,2,2-trifluoroethylthio group;
a C6-10 arylthio group such as a phenylthio group or a naphthylthio group;
a 5 to 6-membered heteroarylthio group such as a thiazolylthio group or a pyridylthio group;
a C1-6 alkylsulfinyl group such as a methylsulfinyl group, an ethylsulfinyl group or a t-butylsulfinyl group;
a C1-6 haloalkylsulfinyl group such as a trifluoromethylsulfinyl group or a 2,2,2-trifluoroethylsulfinyl group;
a C6-10 arylsulfinyl group such as a phenylsulfinyl group;
a 5 to 6-membered heteroarylsulfinyl group such as a thiazolylsulfinyl group or a pyridylsulfinyl group;
a C1-6 alkylsulfonyl group such as a methylsulfonyl group, an ethylsulfonyl group or a t-butylsulfonyl group;
a C1-6 haloalkylsulfonyl group such as a trifluoromethylsulfonyl group or a 2,2,2-trifluoroethylsulfonyl group;
a C6-10 arylsulfonyl group such as a phenylsulfonyl group;
a 5 to 6-membered heteroarylsulfonyl group such as a thiazolylsulfonyl group or a pyridylsulfonyl group;
a C1-6 alkylsulfonyloxy group such as a methylsulfonyloxy group, an ethylsulfonyloxy group or a t-butylsulfonyloxy group;
a C1-6 haloalkylsulfonyloxy group such as a trifluoromethylsulfonyloxy group or a 2,2,2-trifluoroethylsulfonyloxy group;
a tri-C1-6 alkyl-substituted silyl group such as a trimethylsilyl group, triethylsilyl group or a t-butyldimethylsilyl group;
   a tri-C6-10 aryl-substituted silyl group such as a triphenylsilyl group;
a cyano group; and
a nitro group.

In each of these "substituents", any hydrogen atom in the substituent may be also substituted with a substituent having a different structure. Such examples of the " substituent having a different structure" can include a C1-6 alkyl group, a C1-6 haloalkyl group, a C1-6 alkoxy group, a C1-6 haloalkoxy group, a halogeno group, a cyano group, a nitro group, a hydroxy group, a carboxy group or an amino group.

The above-described "3 to 6-membered heterocyclyl group" contains, as a constituent atom of a ring, 1 to 4 heteroatoms selected from the group consisting of a nitrogen atom, an oxygen atom and a sulfur atom. The heterocyclyl group may be either monocyclic or polycyclic. As long as at least one ring in the polycyclic heterocyclyl group is a heterocyclic ring, the other ring(s) in the polycyclic heterocyclyl group may be any of a saturated alicyclic ring, an unsaturated alicyclic ring or an aromatic hydrocarbon ring. Examples of the "3 to 6-membered heterocyclyl group" can include a 3 to 6-membered saturated heterocyclyl group, a 5 to 6-membered heteroaryl group and a 5 to 6-membered partially unsaturated heterocyclyl group.

Examples of the 3 to 6-membered saturated heterocyclyl group can include an aziridinyl group, an epoxy group, a pyrrolidinyl group, a tetrahydrofuranyl group, a thiazolidinyl group, a piperidyl group, a piperazinyl group, a morpholinyl group, a dioxolanyl group and a dioxanyl group.

Examples of the 5-membered heteroaryl group can include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered heteroaryl group can include a pyridyl group, a pyrazinyl group, a pyrimidinyl group, a pyridazinyl group and a triazinyl group.

However, in the multivalent linking group shown by Z, the substituent of a skeleton which constitutes a mother nucleus of the multivalent linking group does not include a carboxy group.

The divalent to tetravalent linking group derived from an alkane shown by Z is a groups in which any two to four hydrogen atoms on a carbon constituting the alkane of a mother nucleus are converted into a bonding hand, and specific examples thereof can include the linking groups shown below. The bonding hand means a state in which an electron capable of forming a bonding with another functional group is located, and "-" described on a carbon is a bonding hand. Which bonding hand is bonded to a carboxylate ionic group and a benzene ring of triptycene can be arbitrarily determined. The same applies hereinafter.

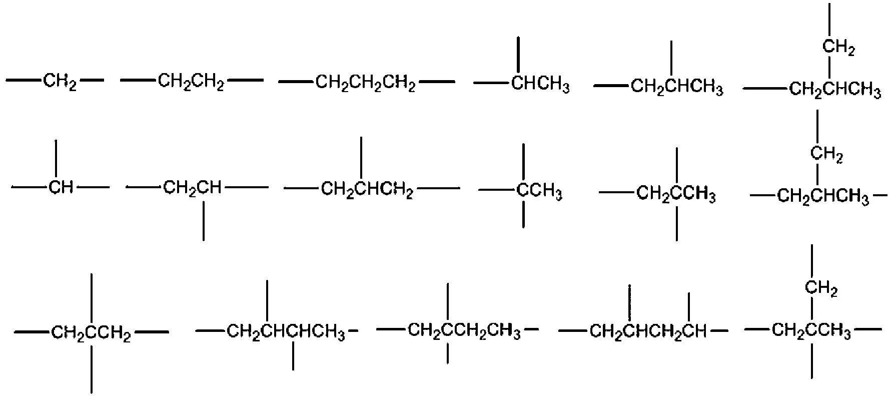

The divalent to tetravalent linking group derived from ethene shown by Z is a group in which any two to four hydrogen atoms on a carbon constituting the ethene of a mother nucleus are converted into a bonding hand, and specific examples thereof can include the linking groups shown below.

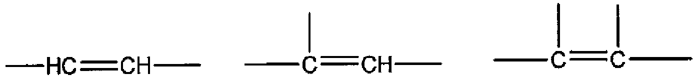

The divalent to tetravalent linking group derived from an aryl shown by Z is a group in which any two to four hydrogen atoms on a carbon constituting the aryl of a mother nucleus are converted into a bonding hand, and specific examples thereof can include the linking groups shown below.

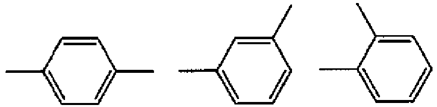

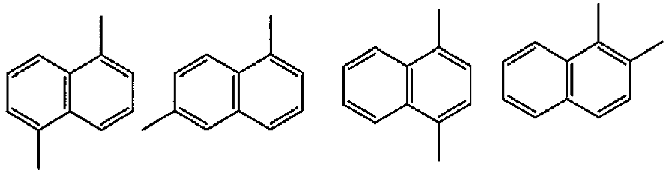

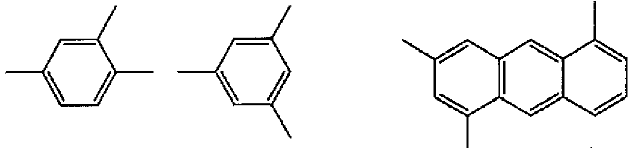

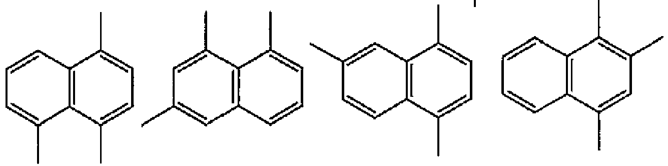

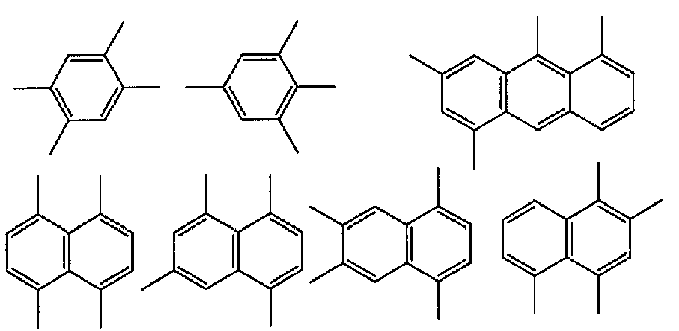

The divalent to tetravalent linking group derived from an heteroaryl shown by Z is a group in which any two to four hydrogen atoms on a carbon constituting the heteroaryl of a mother nucleus are converted into a bonding hand, and specific examples thereof can include the linking groups shown below.

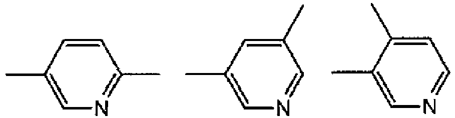

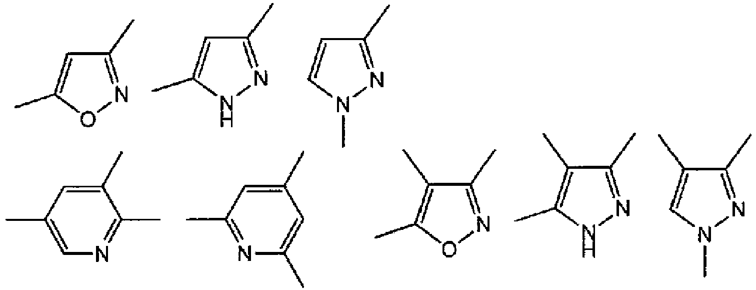

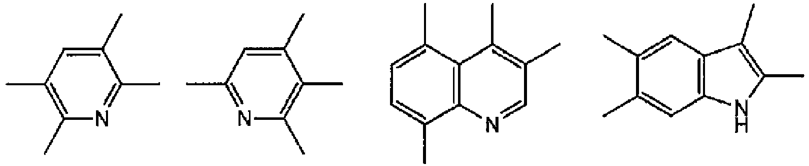

The combination of linking groups of the linking group A group shown by Z means a combination of two or more linking groups selected within a chemically acceptable range from the group consisting of divalent to tetravalent linking groups derived from an alkane, divalent to tetravalent linking groups derived from ethene; an ethynylene group, divalent to tetravalent linking groups derived from an aryl and divalent to tetravalent linking groups derived from a heteroaryl, which are bound by a bonding hand to each other to constitute a multivalent linking group as a whole.

When the liking groups are combined as described above, the valence of each of the linking group is not particularly limited but preferably divalent or trivalent. Specific examples of such a combination can include linking groups shown below.

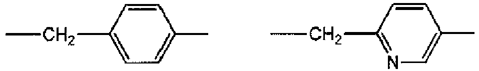

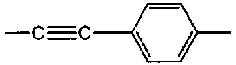

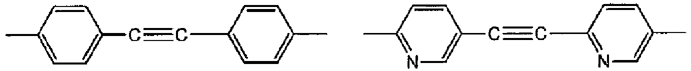

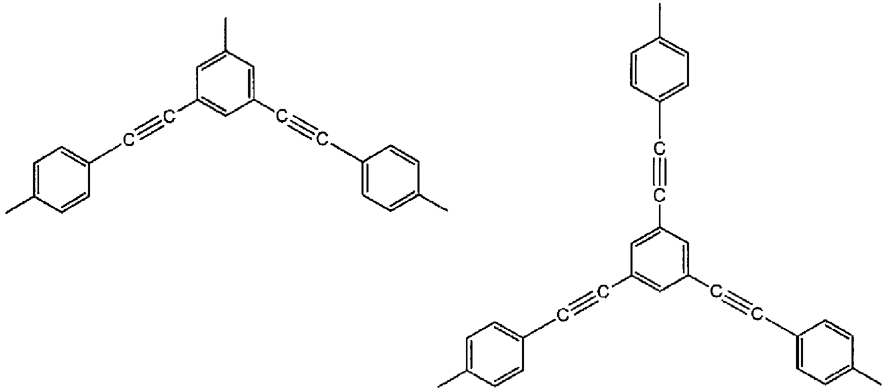

In formulae [IV-1] to [IV-3], R²¹ and R²² each independently represent a hydrogen atom, a C1-6 alkyl group, a C6-10 aryl group, a C1-6 alkylcarbonyl group or a C6-10 arylcarbonyl group.

The "C1-6 alkyl group" may be linear or branched. Examples of the "C1-6 alkyl group" can include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group and an i-hexyl group.

The "C6-10 aryl group" may be either monocyclic or polycyclic. As long as at least one ring in the polycyclic aryl group is an aromatic ring, the other ring(s) in the polycyclic aryl group may be any of a saturated alicyclic ring, an unsaturated alicyclic ring or an aromatic ring.

Examples of the "C6-10 aryl group" can include a phenyl group, a naphthyl group, an azulenyl group, an indenyl group, an indanyl group and a tetralinyl group.

Examples of the "C1-6 alkylcarbonyl group" can include an acetyl group and a propionyl group.

Examples of the "C6-10 arylcarbonyl group" can include a benzoyl group and a 1-naphthylcarbonyl group.

Specific examples of the combinations of the linking groups of the linking group B group can include the linking groups shown below.

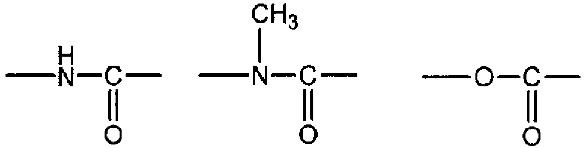

Specific examples of the combination of the linking group selected from the linking group A group and the linking group selected from the linking group B group can include the linking groups shown below.

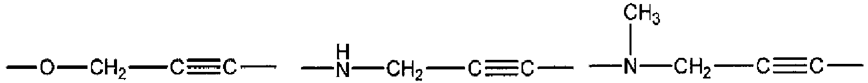

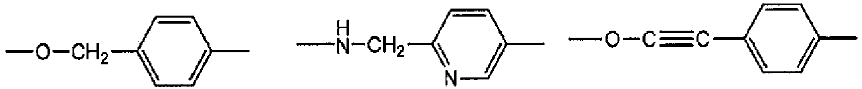

Among the functional group of formula [II], in particular, the functional group of formula [V-1] or formula [V-2] can be preferably exemplified.

In formula [V-2], R³¹ is a C1 to 6 alkyl group, a C3 to 8 cycloalkyl group, a C6 to 10 aryl group, a 3 to 6-membered heterocyclyl group, a C1 to 6 alkoxy group, a C6 to 10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [VI].

Examples of the "C1 to 6 alkyl group" and "C6 to 10 aryl group" can include the same ones as the specific examples exemplified for R²¹.

Examples of the a "C3-8 cycloalkyl group", a "3 to 6 membered heterocyclyl group", a "C1-6 alkoxy group", a "C6-10 aryloxy group", a "heteroaryloxy group", a "halogeno group", a "C1-6 haloalkyl group, a "C1-6 haloalkoxy group", a "C1-6 alkylthio group", a "C6-10 arylthio group", a "heteroarylthio group", a "C1-6 alkylsulfinyl group", a "C6-10 arylsulfinyl group", a "heteroarylsulfinyl group", a "C1-6 alkylsulfonyl group", a "C6-10 arylsulfonyl group" and a "heteroarylsulfonyl group" can include the same ones as the specific examples exemplified for the "substituent".

Examples of the "C6-10 haloaryl group" can include a 4-chlorophenyl group, a 4-fluorophenyl group and a 2,4-dichlorophenyl group.

In formula [VI], R⁴¹ and R⁴² each independently represent a hydrogen atom, a C1-6 alkyl group, a C6-10 aryl group, a C1-6 alkylcarbonyl group or a C6-10 arylcarbonyl group, and specific examples thereof can include the same ones as the specific examples exemplified for R²¹.

Specific examples of formula [VI] can include an amino group; a C1-6 alkyl-substituted amino group such as a methylamino group, dimethylamino group or a diethylamino group; a C6-10 arylamino group such as an anilino group or a naphthylamino group; a C1-6 alkylcarbonylamino group such as an acetylamino group, a propanoylamino group, a butyrylamino group or an i-propylcarbonylamino group; and a benzoylamino group.

Examples of the group of formula [V-2] can include the functional groups shown below.

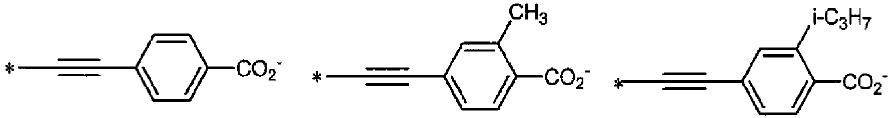

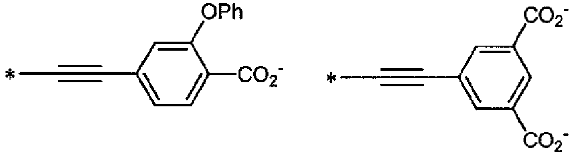

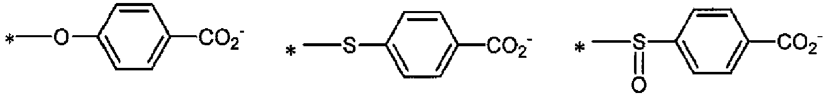

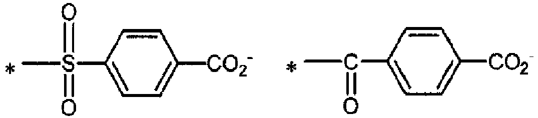

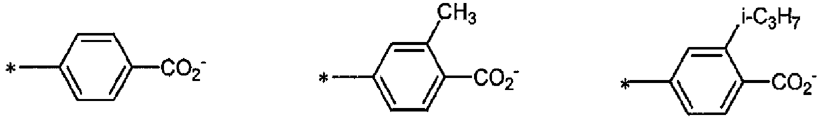

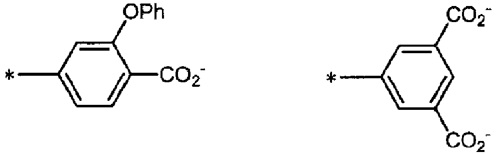

In formula [I], R¹ to R³ each independently represent a hydrogen atom, a C1-6 alkyl group, a C3-8 cycloalkyl group, a C6-10 aryl group, a 3 to 6-membered heterocyclyl group, a C1-6 alkoxy group, a C6-10 aryloxy group, a heteroaryloxy group, a halogeno group, C1-6 haloalkyl group, a C6-10 haloaryl group, a C1-6 haloalkoxy group, C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [III].

In formula [III], R¹¹ and R¹² each independently represent a hydrogen atom, a C1-6 alkyl group, a C6-10 aryl group, a C1-6 alkylcarbonyl group or a C6-10 arylcarbonyl group.

Specific examples of R¹ to R³ can include the same ones as the specific examples exemplified for R³¹, the specific examples exemplified for the "substituent" and the specific examples exemplified for formula [VI].

In formula [I], Y¹ and Y² each independently represent a hydrogen atom, a halogeno group, a C1-6 alkyl group or a C1-6 alkoxy group, and specific examples thereof can include the same ones as the specific examples exemplified for R²¹ and the specific examples exemplified for the "substituent". However, when the multivalent metal ion is a trivalent metal ion, Y¹ and Y² each independently represent a halogeno group, a C1-6 alkyl group or a C1-6 alkoxy group.

Specific examples of the carboxylate ion of formula [I] can include the following structures:

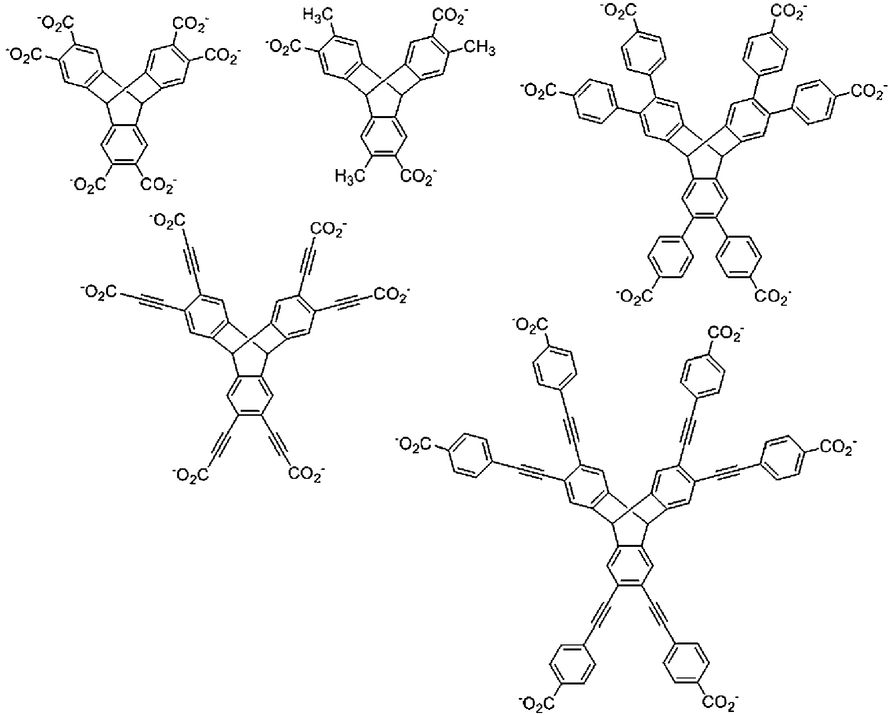

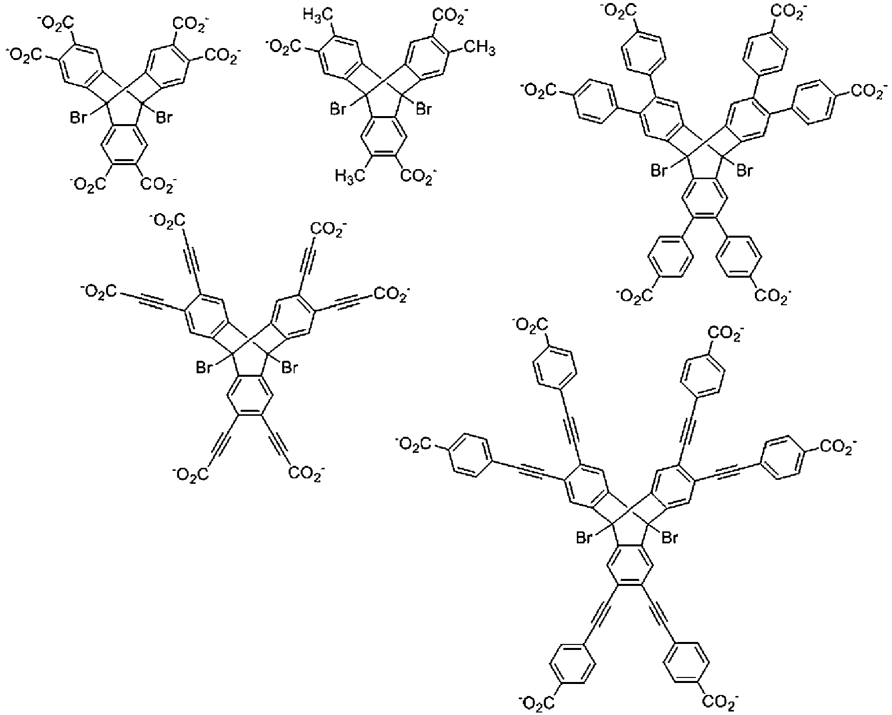

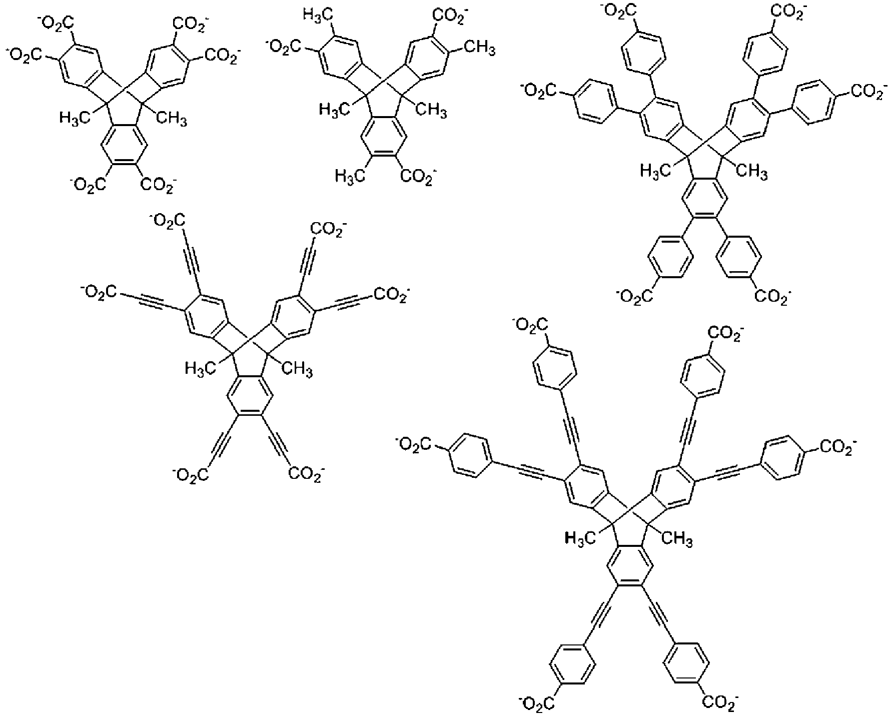

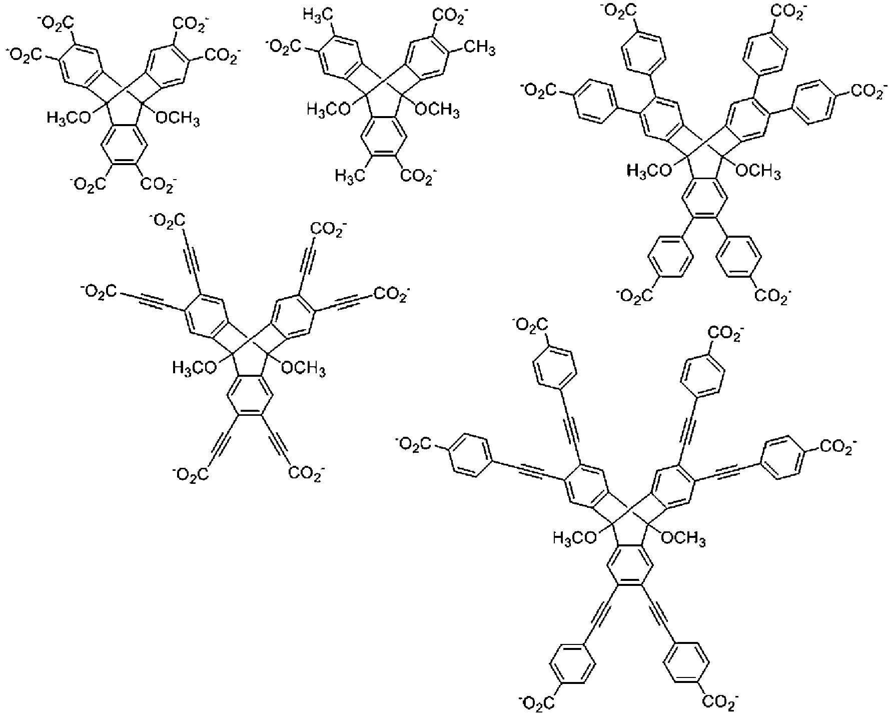

The metal-organic framework of the present invention can include an organic ligand other than the organic ligand that is a carboxylate ion of formula [I] (hereinafter referred to as the organic ligand of formula [I]). Specific examples of such an organic ligand can include terephthalic acid, phthalic acid, isophthalic acid, 5-cyanoisophthalic acid, 1,3,5-trimesic acid, 1,3,5-tris(4-carboxyphenyl)benzene, 1,3,5-trimesic acid, 4, 4'-dicarboxybiphenyl, 3,5-dicarboxypyridine, 2,4-dicarboxypyrazine, 1,3,5-tris(4-carboxyphenyl)benzene, 1,2,4,5-tetrakis(4-carboxyphenyl)benzene, 9, 10-anthracenedicarboxylic acid, 2,6-naphthalenedicarboxylic acid, [1,1':4',1"]terphenyl-3,3",5,5"-tetracarboxylic acid, biphenyl-3,3",5,5"-tetracarboxylic acid, 3,3',5,5'-tetracarboxydiphenylmethane, 1,3,5-tris(4'-carboxy[1,1'-biphenyl]-4-yl)benzene, 1,3,5-tris(4-carboxyphenyl)triazine, 1,2-bis(4-carboxy-3-nitrophenyl)ethene, 1,2-bis(4-carboxy-3-aminophenyl)ethene, trans,trans-muconic acid, fumaric acid, benzimidazole, imidazole, 1,4-diazabicyclo[2.2.2]octane (DABCO), pyrazine, 4,4'-dipyridyl, 1,2-di(4-pyridyl)ethylene, 2,7-diazapyrene, 4,4'-azobispyridine and bis(3-(4-pyridyl)-2,4-pentandionato)copper.

When the organic ligand of formula [I] and the organic ligand other than one of formula [I] are mixed and used, the mixing molar ratio is not particularly limited. However, in the case of a pillar molecule such as a pillar molecule that causes crosslinking to construct a three-dimensional structure such as a pillared-layer type structure, the organic ligand other than one of formula [I] is preferably used in an excessive amount relative to the organic ligand of formula [I].

Examples of the method for producing a metal-organic framework of the present invention that can be used include, but not limited to: a solution method such as a solvent diffusion method, a solvent agitation method or a hydrothermal method; a microwave method in which a reaction solution is irradiated with microwaves to uniformly heat the entire system in a short time; an ultrasonic method, in which a reaction vessel is irradiated with ultrasonic waves to repeatedly cause a change in pressure in the reaction vessel, leading to a phenomenon, referred to as cavitation, that a solvent forms bubbles and they collapse and in which a high energy field of about 5,000 K and 10,000 bar is locally formed and becomes a reaction field for nucleation of crystal; a solid-phase synthesis method in which a metal ion source and an organic ligand are mixed without using any solvent; and a liquid assisted grinding (LAG) method in which a metal ion source is mixed with an organic ligand with water added in an amount comparable to water of crystallization.

The method for producing a metal-organic framework of the present invention comprises, for example, steps of preparing a first solution containing a metal compound as a metal ion source and a solvent, a second solution containing a carboxylic acid as a raw material for formula [I] and a solvent, and optionally, a third solution containing another organic ligand and a solvent, respectively, and a step of mixing the first solution, the second solution and the third solution to prepare a reaction liquid and heating it to obtain an metal-organic framework. The first to third solutions do not need to be prepared separately. For example, the above metal compound, the carboxylic acid as a raw material for formula [I], another organic ligand and the solvent may be mixed at once to prepare one solution.

The mixing molar ratio of the above metal compound, the carboxylic acid as a raw material for formula [I] and optionally another organic ligand can be any ratio selected depending on the pore size and surface characteristics of the obtained metal-organic framework, but the metal compound is preferably used in an amount of 2 mol or more and more preferably 3 mol or more relative to the carboxylic acid as a raw material for formula [I] and optionally another organic ligand.

The concentration of the above metal ion in the reaction liquid is preferably in the range of 25 to 200 mol/L.

The concentration of the organic ligand of formula [I] in the reaction liquid is preferably in the range of 10 to 100 mol/L.

The concentration of the organic ligand other than the organic ligand of formula [I] in the reaction liquid is preferably in the range of 25 to 100 mol/L.

The solvent to be used can be one or more solvent selected from the group consisting of N, N-dimethylformamide (hereinafter referred to as "DMF"), N, N-diethylformamide (hereinafter referred to as "DEF"), N,N-dimethylacetamide and water. Among them, preferred is the use of DMF, DEF or dimethylacetamide alone or alternatively the use of a DMF/water mixed solvent, a DEF/water mixed solvent or an N, N-dimethylacetamide/water mixed solvent.

An acid component such as formic acid or acetic acid can be also optionally added.

The heating temperature of the reaction liquid is preferably 80°C or more and more preferably 100 to 140°C. A reaction temperature of 100°C or more tends to easily produce the metal-organic framework of interest. In contrast, a reaction temperature of 140°C or less does not easily decompose a solvent such as DMF or DEF.

The method for storing a gas using a metal-organic framework of the present invention is, but not particularly limited to, preferably a method comprising a step of contacting the metal-organic framework of the present invention with a gas to cause the gas to be adsorbed inside the metal-organic framework, and the contacting manner is not particularly limited. Examples of the method for storing a gas include: a method in which a tank is filled with a gaseous metal-organic framework of the present invention to form a gas storage tank and the gas is allowed to flow into the tank; a method in which the metal-organic framework of the present invention is supported on the surface constituting the inner wall of the tank to form a gas storage tank and the gas is allowed to flow into the tank; and a method in which a tank is formed of a material containing the metal-organic framework of the present invention as a gas storage tank and the gas is allowed to flow into the tank.

Hereinafter, the present invention will be described in more detail with reference to Examples, but the present invention is not limited thereto.

### Examples

### Example 1

### (Production of metal-organic framework)

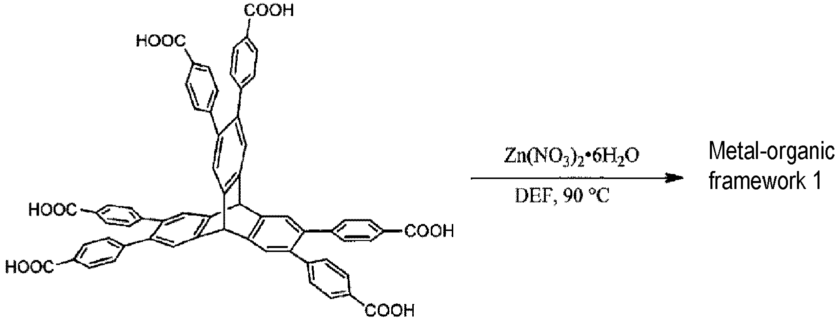

4,4′4ʺ4‴4ʺʺ4ʺ‴-(9,10-Dihydro-9,10-[1,2]b enzenoanthracene-2,3,6,7,14,15-hexayl)hexabenzoic acid (hereinafter referred to as "Organic ligand 1") (101 mg) and zinc nitrate hexahydrate (190 mg) were dissolved in DEF (2 mL) . The mixture was stirred at room temperature for 10 minutes and filtered, and the solution was then placed in a screw-capped vial, sealed and heated at 90°C for 24 hours. The solution was cooled to room temperature, and the resulting crystal was filtered off and washed with DEF and then with chloroform. The crystal was immersed in chloroform for 24 hours. The solid left after filtration was dried in vacuum to obtain Metal-organic framework 1 (61.4 mg).

### Example 2

Metal-organic framework 2 (92.0 mg) was obtained in the same manner as in Example 1 except that the heating temperature was 120°C instead of 90°C.

### Example 3

Metal-organic framework 3 (75.3 mg) was obtained in the same manner as in Example 1 except that DMF was used instead of DEF.

### Example 4

Metal-organic framework 4 (97.9 mg) was obtained in the same manner as in Example 2 except that DMF was used instead of DEF.

### Example 5

A mixed solution of DMF (0.8 mL) and triethylamine (0.05 mL) was added to Organic ligand 1 (61.6mg) in a 10 mL centrifuge tube and was stirred at room temperature for 15 minutes to provide Solution 1. DMF (1.0 mL) was added to zinc acetate dihydrate (104.5 mg) in another 10 mL centrifuge tube and was stirred at room temperature for 15 minutes to provide Solution 2. Solution 2 was added to Solution 1 and stirred at room temperature for 2.5 hours. DMF (5.0 mL) was added thereto and the mixture was centrifuged. It was decanted and was then immersed in DMF. On the next day, the mixture was centrifuged, decanted and then washed with chloroform. The mixture was centrifuged, decanted and then immersed in chloroform for 48 hours. The mixture was centrifuged, decanted and then immersed in chloroform for 48 hours. The mixture was centrifuged, decanted and then immersed in chloroform for 72 hours. The mixture was centrifuged, decanted and then dried under reduced pressure at 150°C for 6 hours to obtain Metal-organic framework 5 (78.3 mg).

### Example 6

Organic ligand 1 (100.9 mg) and zirconium oxychloride octahydrate (102.2 mg) were dissolved in DMF (5.2 mL) in a 20 mL vial. Formic acid (0.7 mL) was further added thereto. It was heated in an oven at 120°C for 24 hours. After cooling it to room temperature, the operation of washing it with DMF and centrifuging and decanting it was carried out three times. The operation of immersing it in DMF for 24 hours and centrifuging and decanting it was carried out three times. The operation of immersing it in methanol for 24 hours and centrifuging and decanting it was carried out three times, and drying was then carried out under reduced pressure at 150°C for 6 hours to obtain Metal-organic framework 6 (127.9 mg).

### Example 7

DMF was added in an amount of 2.1 mL to Organic ligand 1 (101.6 mg), zinc nitrate hexahydrate (93.3 mg) and 1,4-diazabicyclo[2,2,2]octane (DABCO) (17.0 mg), and stirred at room temperature for 10 minutes. It was filtered, then transferred to an autoclave and heated in an oven at 120°C for 48 hours. It was cooled to room temperature and decanted. DMF was added thereto in an amount of 10 mL and stirred at room temperature for 30 minutes, and the mixture was then allowed to be immersed in DMF for 24 hours. After decantation, 10 mL of chloroform was added thereto and the mixture was stirred at room temperature for 30 minutes. It was decanted and was then immersed in chloroform for 48 hours. The mixture was centrifuged, decanted and then dried under reduced pressure at 150°C for 6 hours to obtain Metal-organic framework 7 (73.6 mg) .

### Example 8

Organic ligand 1 (100.3 mg) and ethanol (0.34 mL) were added to a 50 mL eggplant flask to provide Solution 3. Copper nitrate hemipentahydrate (142.5mg) was dissolved in water (4.1 mL) in another 50 mL eggplant flask to provide Solution 4. Solution 4 was added to Solution 3, and the obtained solution was transferred to an autoclave and heated in an oven at 140°C for 24 hours. After cooling it to room temperature, the mixture was centrifuged and decanted. The operation of washing it with water and centrifuging and decanting it was carried out three times. The operation of washing it with ethanol and centrifuging and decanting it was carried out three times, and drying was then carried out under reduced pressure at 150°C for 6 hours to obtain Metal-organic framework 8 (62.1 mg).

### Example 9

Organic ligand 1 (100.4 mg, 0.103 mmol) and magnesium nitrate hexahydrate (79.4 mg, 0.310 mmol) were added to an autoclave, and a mixed solvent (2.5 mL) of tetrahydrofuran (hereinafter referred to as "THF") : water: a 1 M aqueous sodium hydroxide solution = 7: 3: 2 was added thereto. It was heated in an oven at 100°C for 24 hours. After cooling it to room temperature, the mixture was centrifuged and decanted. The operation of washing it with DMF and centrifuging and decanting it was carried out three times. It was washed with chloroform and subjected to filtration under pressure. It was immersed in chloroform for 24 hours, filtered under pressure and then dried under reduced pressure at 150°C for 6 hours to obtain Metal-organic framework 9 (40.2 mg) .

### Example 10

Copper nitrate trihydrate (73.4 mg) and Organic ligand 1 (101.7 mg) were dissolved in DMF (2.1 mL) . The mixture was stirred at room temperature for 10 minutes and filtered, and the resulting solution was then placed in a screw-capped vial, sealed and heated at 90°C for 36 hours. It was cooled to room temperature, DMF was added thereto, and the mixture was centrifuged and decanted. Chloroform was added thereto, and the mixture was centrifuged and decanted, and then immersed in chloroform for 24 hours. After centrifuging and decanting it, the resulting solid was dried under vacuum to obtain Metal-organic framework 10 (97.2 mg).

### Example 11

Metal-organic framework 11 (72.6 mg) was obtained in the same manner as in Example 10 except that the heating temperature was 120°C instead of 90°C.

### Example 12

Metal-organic framework 12 (78.3 mg) was obtained in the same manner as in Example 10 except that DEF was used instead of DMF.

### Example 13

Metal-organic framework 13 (92.8 mg) was obtained in the same manner as in Example 11 except that DEF was used instead of DMF.

### [Reference Example 1]

Production of 4,4′,4ʺ,4‴,4ʺʺ,4ʺ‴-(9,10-dibromo-9,10-dihydro-9,10 -[1,2]benzenoanthracene-2,3,6,7,14,15-hexayl)hexabenzoic acid (hereinafter referred to as "Organic ligand 2")

### (Step 1) Synthesis of 2,3,6,7,9,10,14,15-octabromo-9,10-dihydro-9,10[1',2']-benz enoanthracene

To a two-necked flask were added 9,10-dibromo-9,10-dihydro-9,10[1',2']-benzenoanthracene (0.504 g) , iron powder (80.6mg) and 1, 2-dichloroethane (6 mL), followed by slow dropwise addition of a mixed solution of bromine (0.37 mL) and 1,2-dichloroethane (6 mL) . The mixture was stirred to at 80°C for 1 hour, then was returned to room temperature and quenched with an aqueous sodium thiosulfate solution, followed by suction filtration to obtain 2,3,6,7,9,10,14,15-octabromo-9,10-dihydro-9,10[1',2']-benz enoanthracene (0.574 g) as an off-white solid.

### (Step 2) Synthesis of Organic ligand 2

To a Schlenk flask were added 2,3,6,7,9,10,14,15-octabromo-9,10-dihydro-9,10[1',2']-benz enoanthracene (0.502 g) obtained in step 1, cesium carbonate (3.91 g), 4-methoxycarbonylphenylboronic acid (2.13 g), tetrakis(triphenylphosphine)palladium (0.233 g), followed by addition of THF (50 mL) . The mixture was heated under reflux with stirring for two days and returned to room temperature, and the solvent was distilled off under reduced pressure. It was extracted with dichloromethane, dried over anhydrous magnesium sulfate and subjected to natural filtration, and the solvent was then distilled off under reduced pressure with a rotary evaporator. Purification was carried out by silica gel column chromatography to obtain a solid. The obtained solid was dissolved in ethyl acetate, and hexane was then added thereto to precipitate a solid. The obtained solid was transferred to an eggplant flask, potassium hydroxide (0.933 g) was added thereto, a mixed solvent of methanol/THF/water (1:1:1; 150 mL in total) was added thereto, and the mixture was heated under reflux with stirring overnight. It was returned to room temperature, concentrated hydrochloric acid was added thereto, and the organic solvent was then distilled off under reduced pressure. The precipitated solid was subjected to suction filtration to obtain Organic Ligand 2 (0.168 g) as a colorless solid.

### Example 14

Zinc nitrate hexahydrate (66.7 mg) and Organic ligand 2 (41.3 mg) obtained in Reference Example 1 were dissolved in DMF (0.71 mL) . The mixture was stirred at room temperature for 10 minutes and filtered, and the solution was then placed in a screw-capped vial, sealed and heated at 90°C for 48 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 14 (26.0 mg).

### Example 15

Metal-organic framework 15 (30.9 mg) was obtained in the same manner as in Example 14 except that the heating temperature was 120°C instead of 90°C and the heating time was 24 hours.

### Example 16

Metal-organic framework 16 (29.9 mg) was obtained in the same manner as in Example 14 except that DEF was used instead of DMF.

### Example 17

Metal-organic framework 17 (24.0mg) was obtained in the same manner as in Example 15 except that DEF was used instead of DMF.

### Example 18

Organic ligand 2 (90.6 mg, 0.080 mmol), zirconium tetrachloride (111.9 mg, 0.48 mmol) , DMF (6.3 mL), water (0.1 ml, 72eq.) and acetic acid (0.87 g, 180 eq.) were placed in a screw-capped vial and subjected to ultrasonic treatment. Thereafter, it was sealed and heated at 120°C for 24 hours. It was cooled to room temperature, centrifuged and decanted to obtain a solid. The operation of adding DMF to the solid and centrifuging and decanting it was repeated three times. The same operation as above except that the solvent was changed to acetone was repeated three times, and the solid was washed and then immersed in acetone for 24 hours. The mixture was centrifuged, decanted and then dried under vacuum at 150 °C for 6 hours to obtain Metal-organic framework 18 (131.3 mg) .

### (Reference Example 2)

### Synthesis of 4,4′,4ʺ,4‴,4ʺʺ,4ʺ‴-((9,10-dihydro-9,10-[1,2]benzen oanthracene-2,3,6,7,14,15-hexay1)hexakis(ethyn-2,1-diyl))h exabenzoic acid (hereinafter referred to as "Organic ligand 3")

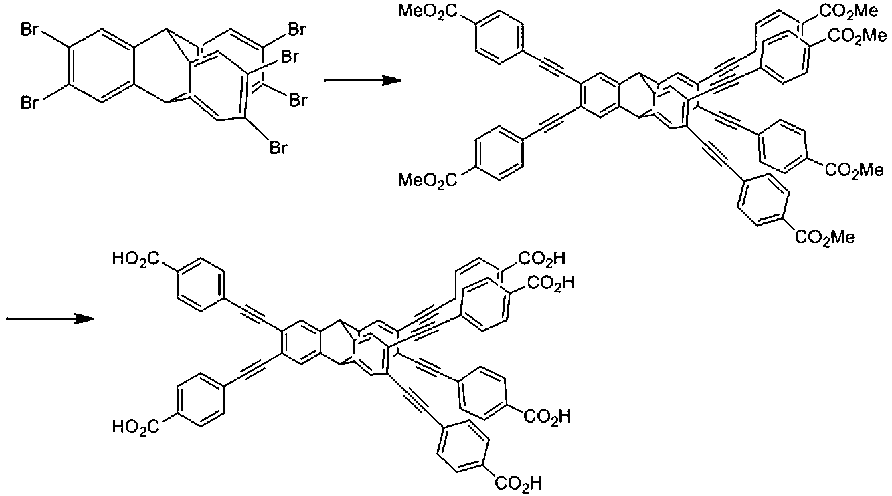

### (Step 1) Synthesis of hexamethyl 4,4′,4ʺ,4‴,4ʺʺ,4ʺ‴-((9,10-dihydro-9,10-[1,2]benzen oanthracene-2,3,6,7,14,15-hexay1)hexakis(ethyn-2,1-diyl))h exabenzoate

To a 100 mL Schlenk flask were added 2,3,6,7,14,15-hexabromo-9,10-dihydro-9,10-[1,2]benzenoanth racene (0.737 g, 1.01 mmol), CuI (43.0 mg, 0.226 mmol), PdCl₂(PPh₃)₂ (98.6 mg, 0.104 mmol), PPh₃ (53.5 mg, 0.204 mmol) and methyl 4-ethynylbenzoate (1.30 g, 8.14 mmol), followed by addition of 22 mL of degassed triethylamine. The mixture was heated under reflux with stirring for two days, and the solvent was then distilled off under reduced pressure. It was dissolved in dichloromethane and washed with a saturated aqueous sodium chloride solution. The organic layer was dried over magnesium sulfate and subjected to filtration, and the solvent was then distilled off under reduced pressure with a rotary evaporator. Purification was carried out by silica gel column chromatography (dichloromethane → ethyl acetate), and the solvent was then distilled off under reduced pressure with a rotary evaporator to obtain a solid. The obtained solid was reprecipitated with ethyl acetate and hexane and subjected to suction filtration to obtain hexamethyl 4,4′,4ʺ,4‴,4ʺʺ,4ʺ‴-((9,10-dihydro-9,10-[1,2]benzen oanthracene-2,3,6,7,14,15-hexay1)hexakis(ethyn-2,1-diyl))h exabenzoate (0.504 g, 0.419 mmol) as a brown solid (yield: 41.5%).

### (Step 2) Synthesis of Organic ligand 3

To a 300 mL eggplant flask were added hexamethyl 4,4′,4ʺ,4‴,4ʺʺ,4ʺ‴-((9,10-dihydro-9,10-[1,2]benzen oanthracene-2,3,6,7,14,15-hexayl)hexakis(ethyn-2,1-diyl))h exabenzoate (0.413g, 0.343 mmol) and potassium hydroxide (1.58 g, 28.1 mmol) followed by addition of a mixed solvent of MeOH, THF and H₂O (1:1:1; 150 mL in total). The mixture was heated under reflux with stirring overnight. It was returned to room temperature, concentrated hydrochloric acid was then added thereto, and the organic solvent was distilled off under reduced pressure. The precipitated solid was subjected to suction filtration to obtain Organic Ligand 3 (0.291 g, 0.260 mmol) as a brown solid (yield: 75.8%).

### (Reference Example 3)

### Synthesis of 4,4′,4ʺ,4‴,4ʺʺ,4ʺ‴-((9,10-dibromo-9,10-dihydro-9,1 0-[1,2]]benzenoanthracene-2,3,6,7,14,15-hexayl)hexakis(eth yn-2,1-diyl))hexabenzoic acid (hereinafter referred to as "Organic ligand 4")

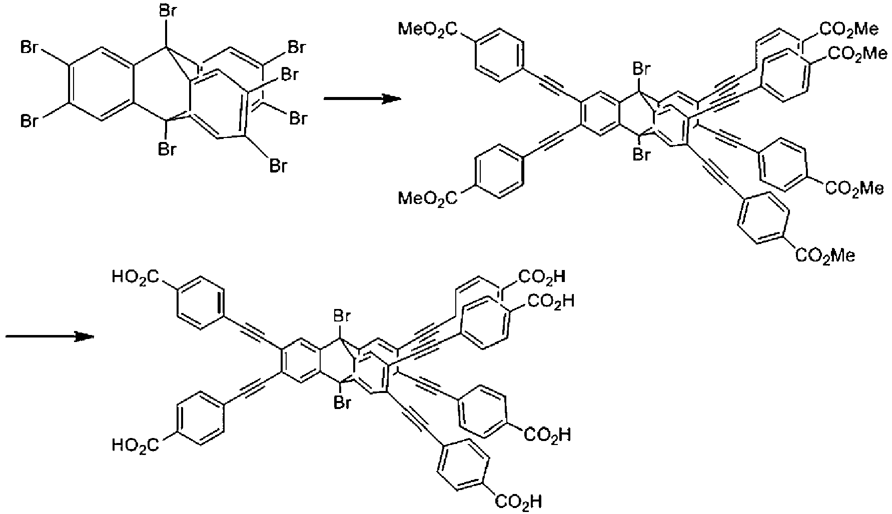

### (Step 1) Synthesis of hexamethyl 4,4′,4ʺ,4‴,4ʺʺ,4ʺ‴-((9,10-dibromo-9,10-dihydro-9,1 0-[1,2]benzenoanthracene-2,3,6,7,14,15-hexayl)hexakis(ethy n-2,1-diyl))hexabenzoate

To a 100 mL Schlenk flask were added 2,3,6,7,9,10,14,15-octabromo-9,10-dihydro-9,10-[1,2]benzen oanthracene (0.917 g, 1.04 mmol), CuI (41.5 mg, 0.278 mmol), PdCl₂(PPh₃)₂ (74.5 mg, 0.106 mmol), PPh₃ (57.3 mg, 0.204 mmol) and methyl 4-ethynylbenzoate (1.30 g, 8.10 mmol), followed by addition of 22 mL of degassed triethylamine. The mixture was heated under reflux with stirring for two days, and the solvent was distilled off under reduced pressure. It was dissolved in dichloromethane and washed with a saturated aqueous sodium chloride solution. The organic layer was dried over magnesium sulfate and subjected to filtration, and the solvent was then distilled off under reduced pressure with a rotary evaporator. Purification was carried out by silica gel column chromatography (dichloromethane → ethyl acetate), and the solvent was then distilled off under reduced pressure with a rotary evaporator to obtain a solid. The obtained solid was reprecipitated with ethyl acetate and hexane and subjected to suction filtration to obtain hexamethyl 4,4′,4ʺ,4‴,4ʺʺ,4ʺ‴-((9,10-dibromo-9,10-dihydro-9,1 0-[1,2]benzenoanthracene-2,3,6,7,14,15-hexayl)hexakis(ethy n-2,1-diyl)) hexabenzoate (1.19 g, 0.847 mmol) as a brown solid (yield: 84.0%).

### (Step 2) Synthesis of Organic ligand 4

To a 300 mL eggplant flask were added hexamethyl 4,4′,4ʺ,4‴,4ʺʺ,4ʺ‴-((9,10-dibromo-9,10-dihydro-9,1 0-[1,2]benzenoanthracene-2,3,6,7,14,15-hexayl)hexakis(ethy n-2,1-diyl))hexabenzoate (0.899 g, 0.660 mmol) and potassium hydroxide (0.767 g, 11.7 mmol), followed by addition of a mixed solvent of MeOH, THF and H₂O (1:1:1; 150 mL in total). The mixture was heated under reflux with stirring overnight. It was returned to room temperature, concentrated hydrochloric acid was then added thereto, and the organic solvent was distilled off under reduced pressure. The precipitated solid was subjected to suction filtration to obtain Organic Ligand 4 (0.767 g, 0.600 mmol) as a brown solid (yield: 90.9%).

### (Reference Example 4)

### Synthesis of 9,10-dibromo-9,10-dihydro-9,10-[1,2Jbenzenoanthracene-2,3, 6,7,14,15-hexacarboxylic acid (hereinafter referred to as "Organic ligand 5")

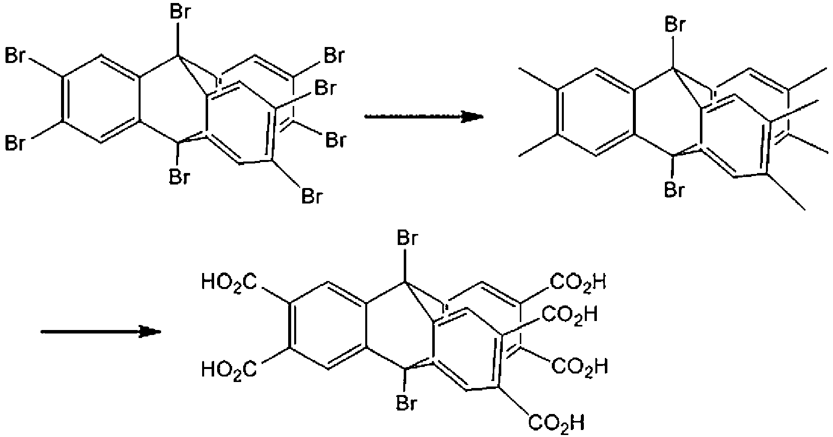

### (Step 1) Synthesis of 9,10-dibromo-2,3,6,7,14,15-hexamethyl-9,10-dihydro-9,10-[1 ,2]benzenoanthracene

To a 100 mL Schlenk flask were added 2,3,6,7,9,10,14,15-octabromo-9,10-dihydro-9,10-[1,2]benzen oanthracene (114 mg, 0.128 mmol) and PdCl₂(PPh₃)₂ (42.6 mg, 0.0608 mmol), followed by addition of 15 mL of dehydrated THF. After stirring at 60°C for 30 minutes, a 1.09 M solution of trimethylaluminum in hexane (1.8 mL, 2.0 mmol) was added thereto and heated under reflux with stirring for 18 hours. It was cooled to 0°C and the reaction was quenched with a 1 M aqueous hydrogen chloride solution. It was extracted with chloroform and washed with a saturated aqueous sodium chloride solution. Thereafter, the organic layer was dried over magnesium sulfate and subjected to filtration, and the solvent was then distilled off under reduced pressure with a rotary evaporator. Purification was carried out by silica gel column chromatography (dichloromethane), and the solvent was then distilled off under reduced pressure with a rotary evaporator to obtain a solid. The obtained solid was washed with methanol and subjected to suction filtration to obtain 9,10-dibromo-2,3,6,7,14,15-hexamethyl-9,10-dihydro-9,10-[1 ,2]benzenoanthracene (42.4 mg, 0.0854) as a colorless solid (yield: 66.7%).

### (Step 2) Synthesis of Organic ligand 5

To a 50 mL two-necked flask were added 9,10-dibromo-2,3,6,7,14,15-hexamethyl-9,10-dihydro-9,10-[1 ,2]benzenoanthracene (1.12 g, 2.26 mmol), potassium permanganate (19.0 g, 120 mmol), 5 mL of pyridine and 5 mL of water. The mixture was heated under reflux with stirring for 24 hours. It was returned to room temperature and filtered through Celite, the residue was washed with hot water, and concentrated hydrochloric acid was added to the filtrates until it became acidic. It was extracted with ethyl acetate and washed with a saturated aqueous sodium chloride solution. Thereafter, the organic layer was dried over magnesium sulfate and subjected to filtration, and the solvent was then distilled off under reduced pressure with a rotary evaporator to obtain Organic ligand 5 (575 mg, 0.851 mmol) as a colorless solid (yield: 37.7%).

### Example 19

Zinc nitrate hexahydrate (98.8 mg) and Organic ligand 3 (60.9 mg) were dissolved in DMF (1.11 mL) . The mixture was stirred at room temperature for 10 minutes and filtered, and the solution was then placed in a screw-capped vial, sealed and heated at 90°C for 48 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 19 (36.0 mg).

### Example 20

Metal-organic framework 20 (38.7 mg) was obtained in the same manner as in Example 19 except that the heating temperature was 120°C instead of 90°C and the heating time was 24 hours.

### Example 21

Metal-organic framework 21 (46.1 mg) was obtained in the same manner as in Example 19 except that DEF was used instead of DMF.

### Example 22

Metal-organic framework 22 (66.1 mg) was obtained in the same manner as in Example 20 except that DEF was used instead of DMF.

### Example 23

Zinc nitrate hexahydrate (143 mg) and Organic ligand 4 (100 mg) were dissolved in DMF (1.6 mL). The mixture was stirred at room temperature for 10 minutes and filtered, and the solution was then placed in a screw-capped vial, sealed and heated at 90°C for 24 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 23 (101 mg) .

### Example 24

Metal-organic framework 24 (103 mg) was obtained in the same manner as in Example 23 except that the heating temperature was 120°C instead of 90°C.

### Example 25

Metal-organic framework 25 (79.4 mg) was obtained in the same manner as in Example 23 except that DEF was used instead of DMF.

### Example 26

Metal-organic framework 26 (125 mg) was obtained in the same manner as in Example 24 except that DEF was used instead of DMF.

### Example 27

Zinc nitrate hexahydrate (266 mg) and Organic ligand 5 (100 mg) were dissolved in DMF (3.0 mL). The mixture was stirred at room temperature for 10 minutes and filtered, and the solution was then placed in a screw-capped vial, sealed and heated at 90°C for 24 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 27 (59.2 mg) .

### Example 28

Metal-organic framework 28 (82.6 mg) was obtained in the same manner as in Example 27 except that the heating temperature was 120°C instead of 90°C.

### Example 29

Metal-organic framework 29 (73.1 mg) was obtained in the same manner as in Example 27 except that DEF was used instead of DMF.

### Example 30

Metal-organic framework 30 (80.9 mg) was obtained in the same manner as in Example 28 except that DEF was used instead of DMF.

### Example 31

Metal-organic framework 31 (78.3 mg) was obtained in the same manner as in Example 18 except that Organic ligand 5 was used instead of Organic ligand 2.

### Example 32

Zinc nitrate hexahydrate (105 mg) and 9,10-dihydro-9,10-[1,2]benzenoanthracene-2,3,6,7,14,15-hex acarboxylic acid (hereinafter referred to as "Organic ligand 6") (93.1 mg) was dissolved in DMF (1.2 mL). The mixture was stirred at room temperature for 10 minutes and filtered, and the solution was then placed in a screw-capped vial, sealed and heated at 90°C for 24 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 32 (27.6 mg).

### Example 33

Metal-organic framework 33 (35.0 mg) was obtained in the same manner as in Example 32 except that the heating temperature was 120°C instead of 90°C.

### Example 34

Metal-organic framework 34 (30.7 mg) was obtained in the same manner as in Example 32 except that DEF was used instead of DMF.

### Example 35

Metal-organic framework 35 (33.2 mg) was obtained in the same manner as in Example 33 except that DEF was used instead of DMF.

### Example 36

Metal-organic framework 36 (94.1 mg) was obtained in the same manner as in Example 18 except that Organic ligand 6 was used instead of Organic ligand 2.

### (Reference Example 5)

### Synthesis of 4,4',4ʺ,4‴,4ʺʺ,4ʺ‴-(9,10-dimethyl-9,10-dihydro-9,1 0-[1,2]benzenoanthracene-2,3,6,7,14,15-hexayl)hexabenzoic acid (hereinafter referred to as "Organic ligand 7")

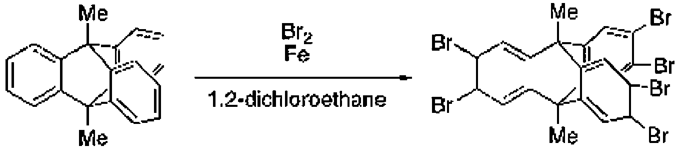

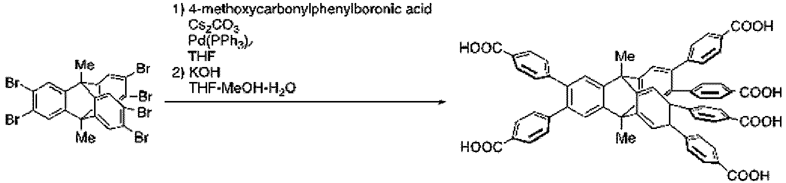

### (Step 1) Synthesis of 9,10-dimethyl-2,3,6,7,14,15-hexabromo-9,10-dihydro-9,10[1' ,2']-benzenoanthracene

Bromine (8.3 mL, 25.6 g, 160 mmol) was added dropwise to a solution of 9,10-dimethyltrypticene (7.48 g, 26.6 mmol) and iron powder (608 mg) in 1,2-dichloroethane solution (270 mL). The mixture was heated and stirred for 10 hours and then returned to room temperature, and the reaction was quenched with an aqueous sodium thiosulfate solution. It was extracted with dichloromethane, the organic layer was dried over anhydrous magnesium sulfate and then filtered, and the solvent was distilled off under reduced pressure. The obtained solid was washed with dichloromethane and filtered to obtain the title compound (20.1 g, 26.6 mmol, quant.) as an off-white solid.

### (Step 2) Synthesis of Organic ligand 7

To a 100 mL Schlenk flask were added under a nitrogen atmosphere 9,10-dimethyl-2,3,6,7,14,15-hexabromo-9,10-dihydro-9,10-[1 ',2']-benzenoanthracene (0.502 g, 0.566 mmol), cesium carbonate (3.91 g, 12.0 mmol), tetrakis(triphenylphosphine)palladium (0.233 g, 0.201 mmol) and 4-methoxycarbonylphenylboronic acid (2.13 g, 11.8 mmol) and dehydrated THF (50 mL), followed by stirring while heating under reflux for two days. The solvent was distilled off under reduced pressure, dichloromethane was then added thereto, and the organic layer was washed with water. The organic layer was washed with brine, then dried over anhydrous magnesium sulfate and filtered. The solvent was distilled off under reduced pressure, purification was carried out by silica gel column chromatography (CH₂Cl₂ → AcOEt), and the solvent was then distilled off under reduced pressure. The obtained solid was mixed with KOH (0.933 g, 16.6 mmol) followed by addition of THF, MeOH and H₂O (50/50/50, 150 mL), and the mixture was heated under reflux with stirring for 18 hours. It was returned to room temperature, concentrated hydrochloric acid was added thereto, and the precipitated solid was subjected to suction filtration to obtain the title compound (0.168 g, 0.148 mmol, 26.1%) as a colorless solid.

### (Reference Example 6) Synthesis of 9,10-dimetyl-9,10-dihydro-9,10-[1,2]benzenoanthracene-2,3, 6,7,14,15-hexacarboxylic acid (hereinafter referred to as "Organic ligand 8")

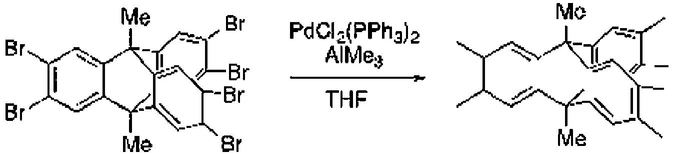

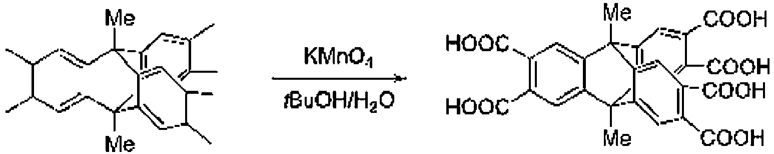

### (Step 1) Synthesis of 2,3,6,7,9,10,14,15-octamethyl-9,10-dihydro-9,10-[1,2]benze noanthracene

A solution of 2,3,6,7,14,15-hexabromo-9,10-dimethyl-9,10-dihydro-9,10-[1 ,2]benzenoanthracene (0.854 g, 1.13 mmol) and PdCl₂(PPh₃)₂ (333 mg, 0.452 mmol) in THF (50 mL) was stirred at 60°C for 30 minutes. A 1.8 M solution of trimethylaluminum in toluene (10.1 mL, 18.1 mmol) was added thereto and heated under reflux with stirring for 18 hours. The temperature was lowered to 0°C, and the reaction was quenched with 1 M hydrochloric acid. It was extracted with chloroform, and the organic layer was washed with brine. The organic layer was dried over anhydrous magnesium sulfate and filtered, and the solvent was then distilled off under reduced pressure. The obtained solid was purified by silica gel column chromatography (CH₂Cl₂:hexane = 1:3), and the solvent was then distilled off under reduced pressure. It was washed with methanol and subjected to suction filtration to obtain the title compound (0.190 g, 0.518 mmol, 45.8%) as a colorless solid.

### (Step 2) Synthesis of Organic ligand 8

2,3,6,7,9,10,14,15-Octamethyl-9,10-dihydro-9,10-[1,2 ]benzenoanthracene (0.154 g, 0.420 mmol) and potassium permanganate (1.17 g, 7.40 mmol) were mixed, followed by addition of a mixed solvent of t-BuOH (2 mL) and H₂O (2 mL) . The mixture was heated with stirring for one day. It was filtered through Celite, and the residue was washed with hot water. The filtrates were mixed, and concentrated hydrochloric acid was added to the mixed filtrates to precipitate a solid. The precipitated solid was subjected to suction filtration to obtain the title compound (59.6 mg, 0.109 mmol, 26.6%) as a colorless solid.

### Example 37

Organic ligand 7 (104 mg, 0.103 mmol) and zinc nitrate hexahydrate (176 mg, 0.592 mmol) were dissolved in DMF (2.0 mL). The mixture was stirred at room temperature for 10 minutes and filtered, and the solution was then placed in a screw-capped vial, sealed and heated at 90°C for 24 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 37 (76.2 mg).

### Example 38

Metal-organic framework 38 (78.6 mg) was obtained as a white crystal in the same manner as in Example 37 except that the heating temperature was 120°C instead of 90°C.

### Example 39

Metal-organic framework 39 (48.5 mg) was obtained as a white crystal in the same manner as in Example 37 except that DEF was used instead of DMF.

### Example 40

Metal-organic framework 40 (64.1 mg) was obtained in the same manner as in Example 38 except that DEF was used instead of DMF.

### Example 41

Metal-organic framework 41 (85.7 mg) was obtained in the same manner as in Example 18 except that Organic ligand 7 was used instead of Organic ligand 2 and 70.5 mg (0.303 mmol, 3.7 eq.) of zirconium tetrachloride was used.

### Example 42

Organic ligand 8 (52.7 mg, 0.0964 mmol) and zinc nitrate hexahydrate (165 mg, 0.554 mmol) were dissolved in DMF (1.8 mL). The mixture was stirred at room temperature for 10 minutes and filtered, and the solution was then placed in a screw-capped vial, sealed and heated at 90°C for 24 hours. It was cooled to room temperature, DMF was added thereto, and the mixture was centrifuged and decanted. Chloroform was added thereto, and the mixture was centrifuged, decanted and then immersed in chloroform for 24 hours. After centrifuging and decanting it, the solid was dried under vacuum to obtain Metal-organic framework 42 (8.0 mg) as a colorless solid.

### Example 43

Metal-organic framework 43 (13.2 mg) was obtained as a colorless solid in the same manner as in Example 42 except that the heating temperature was 120°C instead of 90°C.

### Example 44

Metal-organic framework 44 (2.8 mg) was obtained as a colorless solid in the same manner as in Example 42 except that DEF was used instead of DMF.

### Example 45

Metal-organic framework 45 (58.5 mg) was obtained in the same manner as in Example 43 except that DEF was used instead of DMF.

### Example 46

Metal-organic framework 46 (104.1 mg) was obtained in the same manner as in Example 18 except that Organic ligand 8 was used instead of Organic ligand 2.

### Example 47

Organic ligand 2 (113 mg, 0.10 mmol), chromium nitrate nonahydrate (131 mg, 0.33 mmol) and water (5 ml) were placed in an autoclave, sealed, and heated at 220°C for 24 hours. It was cooled to room temperature, centrifuged and decanted to obtain a solid. The operation of adding water to the solid and centrifuging and decanting it was repeated three times. Ethanol was added in an amount of 20 mL to the solid, and the mixture was heated under reflux for 4 hours, returned to room temperature, then centrifuged and decanted to obtain a solid. The solid was dried under vacuum at 150°C for about 6 hours to obtain Metal-organic framework 47 (41.1 mg).

### Example 48

Metal-organic framework 48 (72.9 mg) was obtained in the same manner as in Example 47 except that Organic ligand 7 was used instead of Organic ligand 2.

### Example 49

Metal-organic framework 49 (27.6 mg) was obtained in the same manner as in Example 47 except that Organic ligand 8 was used instead of Organic ligand 2.

### Example 50

Organic ligand 2 (114 mg, 0.10 mmol), chromium chloride hexahydrate (80 mg, 0.30 mmol) and DMF (10 ml) were completely dissolved by stirring for 10 minutes and then irradiating with ultrasonic waves. The solution was filtered, placed in an autoclave, sealed and heated at 190°C for three days. It was cooled to room temperature, centrifuged and decanted to obtain a solid. The operation of adding DMF to the solid and centrifuging and decanting it was repeated three times. After adding DMF thereto and allowing it to be immersed therein overnight, the solvent was replaced with chloroform, and the similar operation of centrifuging and decanting it was repeated three times. The obtained solid was immersed in chloroform for two days, centrifuged and decanted to obtain a solid. The solid was dried under vacuum at 150°C for about 6 hours to obtain Metal-organic framework 50 (49.5 mg).

### Example 51

Organic ligand 2 (113 mg, 0.10 mmol), aluminum nitrate nonahydrate (113 mg, 0.30 mmol) and water (10 ml) were stirred for 10 minutes, then placed in an autoclave, sealed, and heated at 220°C for 42 hours. It was cooled to room temperature, centrifuged and decanted to obtain a solid. The operation of adding water to the solid and centrifuging and decanting it was repeated three times. After washing with acetone, DMF was added thereto and allowed it to be immersed therein for three days. Thereafter, it was placed in an oven at 100°C and heated for three days. It was returned to room temperature, centrifuged and decanted to obtain a solid. The operation of adding DMF to the solid and centrifuging and decanting it was repeated twice, followed by replacement of the solvent with acetone, centrifugation and decantation. The obtained solid was dried under vacuum at 100°C for about 6 hours to obtain Metal-organic framework 51 (87.1 mg).

### Example 52

Metal-organic framework 52 (67.4 mg) was obtained as a green solid in the same manner as in Example 50 except that Organic ligand 7 was used instead of Organic ligand 2.

### Example 53

Metal-organic framework 53 (60.2 mg) was obtained as a pale-green solid in the same manner as in Example 50 except that Organic ligand 8 was used instead of Organic ligand 2.

### (Elemental analysis test)

Each of Metal-organic frameworks obtained in Examples 1 and 5 to 9 was subjected to an elemental analysis test. The results are shown in Table 1.

**[Table 1]**

| Metal-organic framework No. | Elemental analysis | | |
|---|---|---|---|
| | N(%) | C(%) | H(%) |
| 1 | 0.15 | 59.93 | 2.62 |
| 5 | 2.25 | 60.07 | 3.672 |
| 6 | 0.99 | 51.56 | 3.331 |
| 7 | 0.3 | 56.89 | 3.081 |
| 8 | 0.18 | 63.49 | 3.655 |
| 9 | 0.27 | 71.1 | 4.336 |

### (Measurement of IR spectrum)

IR spectrum was measured for each of Metal-organic frameworks 1 to 9, 14 to 17, 19 to 30, 32 to 35, 37, 40 and 45 obtained in Examples 1 to 9 and 14 to 33. Table 2 shows the peak corresponding to the stretching vibration of the carbonyl group of each Metal-organic framework.

**[Table 2]**

| Metal-organic framework No. | IR |
|---|---|
| | C=O(cm⁻¹) |
| 1 | 1601 |
| 2 | 1601 |
| 3 | 1602 |
| 4 | 1602 |
| 5 | 1602 |
| 6 | 1602 |
| 7 | 1603 |
| 8 | 1604, 1698 |
| 9 | 1607, 1691 |
| 14 | 1603 |
| 15 | 1603 |
| 16 | 1602 |
| 17 | 1602 |
| 19 | 1599 |
| 20 | 1599 |
| 21 | 1599 |
| 22 | 1599 |
| 23 | 1600 |
| 24 | 1599 |
| 25 | 1600 |
| 26 | 1598 |
| 27 | 1599 |
| 28 | 1600 |
| 29 | 1596 |
| 30 | 1604 |
| 32 | 1592 |
| 33 | 1600 |
| 34 | 1606 |
| 35 | 1598 |
| 37 | 1602 |
| 40 | 1602 |
| 45 | 1600 |

### (X-ray structure analysis)

Metal-organic framework 1 obtained in Example 1 was subjected to an X-ray structural analysis under the measurement conditions shown below.

### [Measurement conditions]

One grain of the colorless and transparent crystal of 0.03 × 0.03 × 0.03 mm of Metal-organic framework 1 obtained in Example 1 was placed on a micromount and was subjected to a diffraction experiment using a single crystal X-ray analyzer (D8 VENTURE, manufactured by Bruker). The diffraction data obtained by irradiating a single crystal with an X-ray having a wavelength of 0.78192 Å was analyzed to determine the structure. The results are shown in Table 3.

**[Table 3]**

| | |
|---|---|
| Measurement temperature/°C | -173°C |
| Crystal dimensions/mm | 0.03 × 0.03 × 0.03 |
| Color of crystal | Colorless |
| Chemical formula | C₆₂H₃₂O₁₃Zn₄ |
| Molecular weight | 3795.98 |
| Crystal system | Trigonal (Trigonal system) |
| Space group | *P*-3₁*c* |
| a/Å | 20.068 (3) |
| b/Å | 20.068 (3) |
| c/Å | 22.926 (4) |
| V/Å³ | 7996 (3) |
| Z | 2 |
| Reflections collected | 73199 |
| Independent reflections | 4872 |

### (Measurement of BET specific surface area and measurement of hydrogen storage capacity)

The BET specific surface area and the hydrogen storage capacity at 77 K and atmospheric pressure were measured for Metal-organic frameworks 1 to 37, 40, 41, 45 and 46 obtained in Examples 1 to 37, 40, 41, 45 and 46. The hydrogen storge amount at 298 K and 10 MPa was also measured for Metal-organic framework 1.

The BET specific surface area and the hydrogen storage capacity at 77K and atmospheric pressure were measured with a gas adsorption measurement device, Tristar-II (manufactured by Micromeritics Instrument Corporation), and the hydrogen storage capacity at 298 K and 10 MPa was measured with a gas adsorption measurement device - PTC characterization device (manufactured by SUZUKI SHOKAN Co., Ltd.).

The BET specific surface area was calculated according to the following procedure. About 50 mg of each of Metal-organic frameworks 1 to 33 obtained in Examples 1 to 33 was placed inside a glass cell. The pressure inside the glass cell was reduced to vacuum at a temperature of 135°C and the inside of the glass cell was dried for 6 hours. The glass cell was attached to the gas adsorption measurement device and immersed in a temperature-controlled bath containing liquid nitrogen. The pressure of nitrogen injected into the glass cell was gradually increased. The measurement was carried out until the pressure of nitrogen introduced into the glass cell reached 1.0 × 10⁵ Pa.

The hydrogen storage capacity at 77K and a normal pressure was calculated according to the following procedure. After the measurement for nitrogen, the gas type was changed to hydrogen to carried out the measurement. The pressure of hydrogen injected into the glass cell was gradually increased. The measurement was carried out until the pressure of hydrogen introduced into the glass cell reached 1.0 × 10⁵ Pa.

The hydrogen storage capacity at 298 K and 10 MPa was calculated according to the following procedure. Metal-organic framework 1 obtained in Example 1 was placed in an amount of 220 mg in a stainless-steel sample cell. The pressure inside the cell was reduced to vacuum at a temperature of 135°C, and the temperature and pressure were kept as they were for 65 hours. In this way, the gas and solvent contained inside Metal-organic framework 1 were removed. The cell was immersed in a temperature-controlled bath at 298 K, and with the state kept, the pressure of hydrogen injected into the cell was gradually increased using the gas adsorption measurement device. After the hydrogen pressure inside the cell reached 10 MPa, the pressure of hydrogen contained in the cell was gradually reduced.

The results of the measurements as described above are summarized in Table 4.

**[Table 4]**

| Metal-organic framework No. | BET specific surface area m²_{/}g | Hydrogen storage capacity (w%) | | Color and Property |
|---|---|---|---|---|
| | | 77K-Atmospheric pressure | 298K-10MPa | |
| 1 | 4072 | 1.603 | 2.125 | White powder |
| 2 | 3451 | 1.437 | - | Brown powder |
| 3 | 3639 | 1.478 | - | White powder |
| 4 | 3260 | 1.339 | - | Ocherous powder |
| 5 | 1430 | 1.082 | - | Colorless powder |
| 6 | 665 | 0.959 | - | Pale yellow powder |
| 7 | 2093 | 1.194 | - | Colorless powder |
| 8 | 357 | 0.779 | - | Dark blue-green powder |
| 9 | 967 | 1.680 | - | Colorless powder |
| 10 | 499 | 0.847 | - | Pale blue solid |
| 11 | 740 | 1.014 | - | Pale blue solid |
| 12 | 727 | 0.968 | - | Pale blue solid |
| 13 | 819 | 1.033 | - | Pale blue solid |
| 14 | 2982 | 1.542 | - | Colorless powder |
| 15 | 3104 | 1.503 | - | Colorless powder |
| 16 | 3395 | 1.699 | - | Colorless powder |
| 17 | 3327 | 1.642 | - | Milky white powder |
| 18 | 1475 | 1.289 | - | Colorless solid |
| 19 | 845 | 0.974 | - | Ocherous powder |
| 20 | 1283 | 1.098 | - | Ocherous powder |
| 21 | 79 | 0.256 | - | Ocherous + dark brown powder |
| 22 | 2 | 0.136 | - | Ocherous + dark brown powder |
| 23 | 2 | 0.111 | - | Brown powder |
| 24 | 14 | 0.683 | - | Brown powder |
| 25 | 12 | 0.405 | - | Brown powder |
| 26 | 1 | 0.094 | - | Brown powder |
| 27 | 541 | 1.060 | - | Off-white powder |
| 28 | 596 | 1.148 | - | Off-white powder |
| 29 | 506 | 0.975 | - | Off-white powder |
| 30 | 488 | 0.897 | - | Off-white powder |
| 31 | 518 | 0.6 | - | Pale yellow solid |
| 32 | 130 | 0.344 | - | Dark brown powder |
| 33 | 355 | 0.564 | - | Dark brown powder |
| 34 | 73.8 | 0.375 | - | Yellow powder |
| 35 | 281 | 0.529 | - | Yellow powder |
| 36 | 450 | 0.541 | - | Beige solid |
| 37 | 4034 | 1.922 | - | White crystal |
| 40 | 3447 | 1.657 | - | White crystal |
| 41 | 1398 | 1.355 | - | Colorless solid |
| 45 | 921 | 1.515 | - | Pale yellow solid |
| 46 | 558 | 0.635 | - | Off-white solid |
| 47 | 510 | 0.896 | - | Pale green crystal |
| 48 | 368 | 0.675 | - | Yellow-green powder |
| 49 | 544 | 0.672 | - | Blackish green solid |
| 50 | 2534 | 1.858 | - | Green solid |
| 51 | 797 | 1.274 | - | Pale yellow solid |

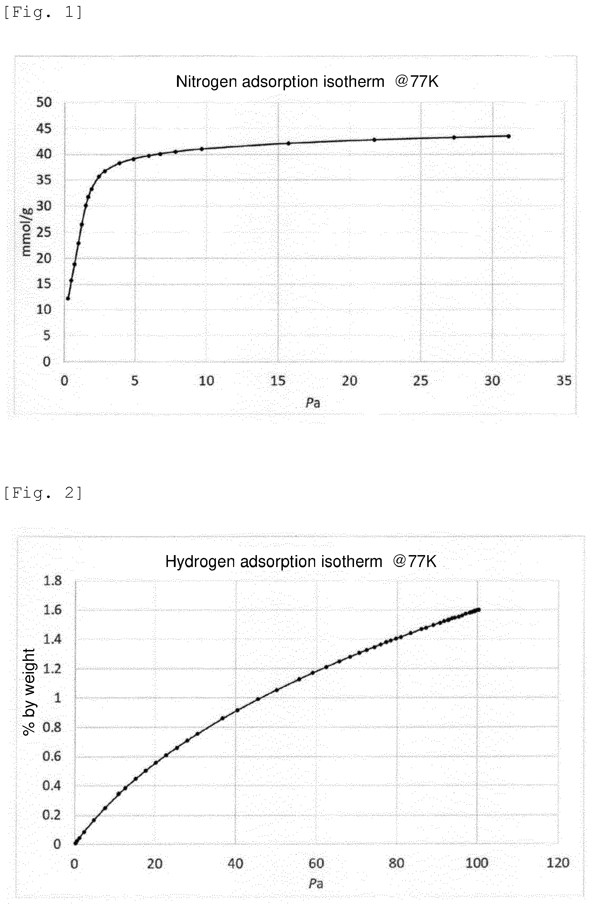

For Metal-organic framework 1 obtained in Example 1, a nitrogen adsorption isotherm (77K) is shown in Figure 1, and a hydrogen adsorption isotherm (77K) is shown in Figure 2.

For Metal-organic frameworks 1, 14 to 16, 37, 40 and 45 obtained in Examples 1, 14 to 16, 37, 40 and 45, the carbon dioxide adsorption at both 273 K and 298 K under an atmospheric pressure and the heat of adsorption of carbon dioxide were measured.

The carbon dioxide adsorption was measured using a gas adsorption measurement device, Tristar-II (manufactured by Micromeritics Instrument Corporation) as follows.

About 50 mg of each of Metal-organic frameworks 1, 14 to 16, 37, 40 and 45 obtained in Examples 1, 14 to 16, 37, 40 and 45 was placed inside a glass cell. The pressure inside the glass cell was reduced to vacuum at a temperature of 135°C and the inside of the glass cell was dried for 6 hours. The glass cell was attached to the gas adsorption measurement device and immersed in a temperature-controlled bath at 273 K or 298 K. The pressure of carbon dioxide injected into the glass cell was gradually increased. The measurement was carried out until the pressure of carbon dioxide introduced into the glass cell reached 1.0 × 10⁵ Pa. The results are shown in Table 5.

**[Table 5]**

| Metal-organic framework No. | Carbon dioxide storage capacity (w%) | | Heat of adsorption of carbon dioxide (kJ/mol) |
|---|---|---|---|
| | 273K-Atmospheric pressure | 298K-Atmospheric pressure | |
| 1 | 10.392 | 0.171 | 14.6 |
| 14 | 8.371 | 0.228 | 32.9 |
| 15 | 7.863 | 0.213 | 34.6 |
| 16 | 9.06 | 0.247 | 35.6 |
| 37 | 11.187 | 6.1 | 17.693 |
| 40 | 9.392 | 4.771 | 19.421 |
| 45 | 18.082 | 11.143 | 21.312 |

### Industrial Applicability

The metal-organic framework of the present invention can adsorb hydrogen and carbon dioxide at a practical level. Consequently, the metal-organic framework can make hydrogen to be utilized more easily, toward the advent of a hydrogen energy-based society, leading to a reduction in carbon dioxide in the atmosphere.

## Claims

1. A metal-organic framework comprising a multivalent metal ion and a carboxylate ion of formula [I]: 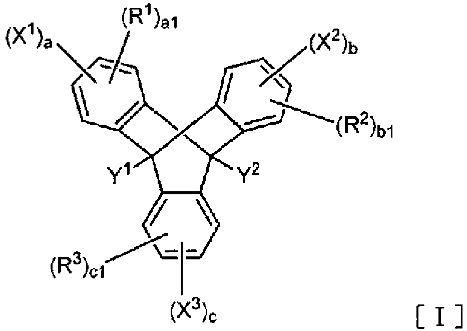 [wherein in formula [I],
X¹ to X³ each independently represent a functional group of formula [II]: 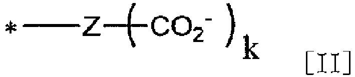 (wherein in formula [II], Z is a single bond or a multivalent linking group, k is an integer of 1 to 4, and * is the position at which a bond is formed with a benzene ring and has also the same meaning in the following);
R¹ to R³ each independently represent a hydrogen atom, a C1-6 alkyl group, a C3-8 cycloalkyl group, a C6-10 aryl group, a 3 to 6-membered heterocyclyl group, a C1-6 alkoxy group, a C6-10 aryloxy group, a heteroaryloxy group, a halogeno group, C1-6 haloalkyl group, a C6-10 haloaryl group, a C1-6 haloalkoxy group, C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [III]: 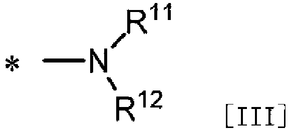 (wherein in formula [III], R¹¹ and R¹² each independently represent a hydrogen atom, a C1-6 alkyl group, a C6-10 aryl group, a C1-6 alkylcarbonyl group or a C6-10 arylcarbonyl group);
a is the number of X¹ and is 0, 1, 2, 3 or 4, and when a is 2 or more, a plurality of X¹s is the same as or different from each other;
a1 is the number of R¹ and is 0, 1, 2, 3 or 4, and when a1 is 2 or more, a plurality of R¹s is the same as or different from each other;
b is the number of X² and is 0, 1, 2, 3 or 4, and when b is 2 or more, a plurality of X²s is the same as or different from each other;
b1 is the number of R² and is 0, 1, 2, 3 or 4, and when b1 is 2 or more, a plurality of R²s is the same as or different from each other;
c is the number of X³ and is 0, 1, 2, 3 or 4, and when c is 2 or more, a plurality of X³s is the same as or different from each other; and
c1 is the number of R³ and is 0, 1, 2, 3 or 4, and when c1 is 2 or more, a plurality of R³s is the same as or different from each other;
provided that a + b + c is 2 or more; and
Y¹ and Y² each independently represent a hydrogen atom, a halogeno group, a C1-6 alkyl group or a C1-6 alkoxy group, provided that when the multivalent metal ion is a trivalent metal ion, Y¹ and Y² each independently represent a halogeno group, a C1-6 alkyl group or a C1-6 alkoxy group];
wherein the carboxylate ion and the multivalent metal ion are bound to each other.

2. The metal-organic framework according to claim 1, wherein the multivalent metal ion is an ion of at least one metal selected from the group consisting of Groups 2 to 13 metals in the periodic table of elements.

3. The metal-organic framework according to claim 1 or 2, wherein the multivalent metal ion is an ion of at least one metal selected from Zn, Fe, Co, Ni, Cu, Zr and Mg.

4. The metal-organic framework according to any one of claims 1 to 3, wherein the multivalent linking group shown by Z in formula [I] is a divalent or trivalent linking group.

5. The metal-organic framework according to any one of claims 1 to 4, wherein the multivalent linking group shown by Z in formula [I] is: a linking group selected from a linking group A group consisting of divalent to tetravalent linking groups derived from an unsubstituted alkane or an alkane having a substituent (except for a carboxy group); divalent to tetravalent linking groups derived from unsubstituted ethene or ethene having a substituent (except for a carboxy group); an ethynylene group; divalent to tetravalent linking groups derived from an unsubstituted aryl or an aryl having a substituent (except for a carboxy group); divalent to tetravalent linking groups derived from an unsubstituted heteroaryl or a heteroaryl having a substituent (except for a carboxy group); and a combination thereof; a linking group selected from a linking group B group consisting of -O-, -S-, -S(O)-, -SO₂-, -C(=O)-, linking groups of following formulae [IV-1] to [IV-3]: 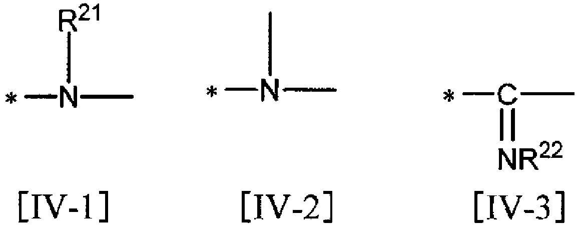 (wherein R²¹ and R²² each independently represent a hydrogen atom, a C1-6 alkyl group, a C6-10 aryl group, a C1-6 alkylcarbonyl group or a C6-10 arylcarbonyl group)
and a combination thereof; or a combination of a linking group selected from the linking group A group and a linking group selected from the linking group B group, provided that the carboxylate ion (CO₂⁻) in formula [II] is bonded to the carbon atom in Z in formula [II].

6. The metal-organic framework according to any one of claims 1 to 5, wherein in formula [I], formula [II] is formula [V-1] or formula [V-2]: 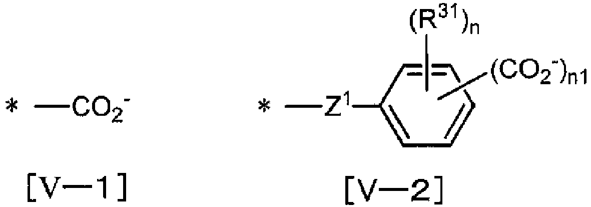 (wherein in formula [V-2],
Z¹ is a single bond, -C=C-, -O-, -S-, -S(O)-, -SO₂- or -C(=O)-;
R³¹ is a C1-6 alkyl group, a C3-8 cycloalkyl group, a C6-10 aryl group, a 3 to 6-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group or a group of formula [VI]: 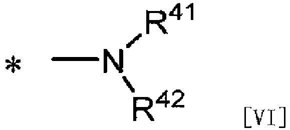 (wherein in formula [VI], R⁴¹ and R⁴² each independently represent a hydrogen atom, a C1-6 alkyl group, a C6-10 aryl group, a C1-6 alkylcarbonyl group or a C6-10 arylcarbonyl group);
n is the number of R³¹ and is 0, 1, 2, 3 or 4, and when n is 2 or more, a plurality of R³¹s is the same as or different from each other; and
n1 is the number of CO₂⁻ and is 1 or 2).

7. The metal-organic framework according to any one of claims 1 to 6, further comprising, as a constituent, an organic ligand other than the carboxylate ion of formula [I].

8. A method for storing a gas, comprising a step of contacting a gas with the metal-organic framework according to any one of claims 1 to 7 to cause the gas to be adsorbed inside the metal-organic framework.
